# EUROPÄISCHE PATENTANMELDUNG

(11) **EP 3 363 465 A1**
(43) Veröffentlichungstag der Anmeldung: **22.08.2018**
(21) Anmeldenummer: 17157280.3
(22) Anmeldetag: 21.02.2017
(51) Int. Cl.: A61K 49/00

(54) **MEMBRANFÄRBEKOMPLEX**

(71) Anmelder: PHARMPUR GmbH, 86343 Königsbrunn (DE)
(72) Erfinder: LIEVENBRÜCK, Melanie, 86343 Königsbrunn (DE); MENZ, Dirk-Henning, 86343 Königsbrunn (DE); MENZ, Helge, 86343 Königsbrunn (DE); MÜLLER, Bernd-Kristof, 86343 Königsbrunn (DE)
(74) Vertreter: Leißler-Gerstl, Gabriele

(57) **Zusammenfassung**

Es wird ein Membranfärbekomplex beschrieben, der mindestens ein Targetingelement [A-L.-Q⁺-Alk B⁻], worin A ein aminohaltiges Ankerelement, L ein Linker, Q⁺ eine quarternäre Aminogruppe, B⁻ ein ophthalmologisch annehmbares Gegenion und Alk eine Alkylkette mit 4 bis 12 Kohlenstoffatomen bedeutet; mindestens ein Chromophor und gegebenenfalls ein Trägermolekül umfasst.

## Beschreibung

Die vorliegende Erfindung betrifft einen Membranfärbekomplex und dessen Verwendung insbesondere in der Ophthalmologie.

Im Bereich der Analytik und Diagnostik sind Farbstoffe zum Anfärben von Geweben, z.B. Membranen, ein wichtiges Hilfsmittel. Ein zum Anfärben geeigneter Farbstoff sollte so selektiv wie möglich an eine bestimmte Art von Gewebe oder Membran binden, sodass die Bindung dieses Farbstoffs ein Hinweis auf das Vorliegen oder für das Auffinden ist. Ein hierfür geeigneter Farbstoff muss allgemein verträglich sein, und sollte eine Farbe haben, die sich von der Umgebung, in der gefärbt werden soll, gut abhebt.

In der Ophthalmologie werden Farbstoffe verwendet, um Membranen anzufärben, die dann chirurgisch entfernt werden sollen. Ein dazu verwendeter Farbstoff sollte wasserlöslich sein, da Lösungsmittel in einem so empfindlichen Organ, wie dem Auge, vermieden werden sollten, sollte eine solche Farbe haben, dass er sich von der Umgebung gut unterscheidet, sollte leicht wieder entfernbar sein und natürlich das Auge nicht reizen oder schädigen. Obwohl es bereits einige Farbstoffe zur Anfärbung von Membranen im Auge gibt, haben alle diese Farbstoffe bisher Nachteile, die mit der vorliegenden Erfindung überwunden werden sollen.

Für Erkrankungen des Auges wie Makuladegeneration, Retinopathie, Netzhautveränderungen etc. ist häufig eine Vitrektomie indiziert, d.h. ein Eingriff, bei dem der Glaskörper oder Teile davon entfernt werden, um dann im Augeninneren die Membrana limitans interna oder gegebenenfalls gebildete epiretinale Membranen von der Netzhaut entfernen zu können, um Heilungsprozesse dadurch zu induzieren, bzw. weitere Schädigungen der Netzhaut zu verhindern. In einer solchen Operation wird die entsprechende Membran mit üblichen chirurgischen Instrumenten von der Netzhaut abgezogen und es ist für den Chirurgen sehr wichtig, dass er zwischen der abzuziehenden Membran und der darunterliegenden Netzhaut unterscheiden kann, um nicht die Netzhaut während des Eingriffs zu schädigen.

Die Retina besteht aus mehreren Schichten mit individuellen Funktionen. Die Aderhaut trägt einen Großteil der Fotorezeptoren und ist zusätzlich für die ausreichende Blutversorgung zuständig. Sie stellt die äußere Begrenzung der Retina dar, im Inneren grenzt die Retina an den Glaskörper. Die Membrana limitans interna ist die Basalmembran der Müller-Zellen, d.h. eine extrazelluläre Membran, die den Glaskörper von der Retina trennt und somit die innere Abgrenzung der Retina darstellt. Dabei ist sie nicht nur mit den Fortsätzen der Müller-Zellen verbunden, sondern ebenfalls über Fibrillen mit dem Glaskörper. Diese Verbindung wird durch Adhäsionen von Kollagenfasern des Glaskörpers mit der ILM geprägt. Die ILM besteht aus verschiedenen Glycoprotein- und Kollagenarten. Ihre Schichtdicke reicht bis zu 2,5 µm. Diese Grenzschicht ist oft von Erkrankungen betroffen, die das Sehvermögen einschränken und durch operative Eingriffe behandelt werden können bzw. müssen.

So können sich beispielsweise sogenannte epiretinale Membranen auf der Netzhaut bilden, die ohne Anlass entstehen können oder durch vorangegangene Erkrankungen oder Eingriffe entstehen. Diese Membranen können sich zusammenziehen, was zu Verformungen der Netzhaut und damit zu Einschränkungen des Sehvermögens führt. Solche Verformungen, die Zug auf die Netzhaut bzw. die Makula ausüben, müssen daher entfernt werden.

Auch für Netzhautablösungen oder das Auftreten von Makuladegenerationen ist die Entfernung der ILM ein Therapieansatz. Um diese feine Membran mit dem "ILM-Peeling" abziehen zu können, ist es nützlich, sie mit einem Farbstoff, der das zu entfernende Gewebe selektiv anfärben kann, zu identifizieren.

Hierzu bekannte Farbstoffe sind z.B. Brillantblau G, Brillantblau R, Patentblau V und Trypanblau sowie Indocyaningrün. Die genannten Farbstoffe zeigen dabei entweder eine toxische Wirkung oder eine zu geringe Färbekraft für den operativen Eingriff.

In der Vergangenheit hat man zur Anfärbung von Membranen Farbstoffe gesucht, die möglichst selektiv an der anzufärbenden Membran haften sollten. Es war daher sehr schwierig, zum spezifischen Anfärben von Membranen jeweils einen geeigneten Farbstoff zu finden, dessen Selektivität in einer ausreichenden Größenordnung liegt bei gleichzeitig physiologischer Annehmbarkeit, Wasserlöslichkeit sowie geeignetem Farbton.

Es war daher Aufgabe der vorliegenden Erfindung, ein Färbemittel zur Verfügung zu stellen, das eine Membran ausreichend färbt, damit sie sichtbar wird. Weiterhin war es Aufgabe der Erfindung, ein Mittel zur Verfügung zu stellen, dessen Farbton durch einfache Substitutionen einstellbar ist. Eine weitere Aufgabe der Erfindung war es, ein Färbemittel bereitzustellen, das nicht in tiefere Zellschichten eindringen kann, andererseits aber selektiv im Zielbereich wechselwirkt, sodass nach dem Anfärben der Farbstoff in ausreichender Menge und ausreichend lang am vorgesehenen Ort bleibt.

All diese Aufgaben werden gelöst mit dem erfindungsgemäßen Membranfärbekomplex, wie er in den Ansprüchen definiert ist.

Gegenstand der Erfindung ist ein Membranfärbekomplex, der mindestens ein Targetingelement [A-L-Q⁺-Alk B⁻], worin A eine Ankergruppe, L ein Linker, Q⁺ eine quaternäre Ammoniumgruppe, B⁻ ein ophthalmologisch annehmbares Gegenion und Alk eine Alkylkette mit einer Kettenlänge von 4 bis 12 Kohlenstoffatomen bedeutet; mindestens ein Chromophor und gegebenenfalls ein Trägermolekül umfasst.

Es wurde nun überraschenderweise gefunden, dass zur Anfärbung von Membranen geeignete Farbstoffe gebildet werden können, indem statt eines Farbstoffs ein Komplex verwendet wird, der mindestens zwei Elemente aufweist, nämlich mindestens ein Targetingelement und mindestens ein Chromophor. Darüber hinaus kann der erfindungsgemäße Membranfärbekomplex als dritte Komponente ein Trägermolekül aufweisen. Mit dieser erfindungsgemäßen Kombination ist es möglich, gezielt Membranen anzusteuern und mit einem Farbstoff, der eine gewünschte Färbung beiträgt, anzufärben.

Die vorteilhaften Eigenschaften werden erzielt, indem für die Funktion des Wechselwirkens mit einer gewünschten Umgebung und für das Färben getrennte Einheiten bereitgestellt werden. Dadurch wird es möglich, ein Chromophor, das einen gewünschten Farbton hat, welches aber nicht selektiv für ein anzufärbendes Gewebe ist, in einer solchen Form bereitzustellen, dass das entsprechende Gewebe selektiv angefärbt werden kann.

### DEFINITIONEN

Der Ausdruck "Komplex", wie er hier verwendet wird, bezeichnet eine Zusammensetzung von Molekülteilen, welche, auch unabhängig voneinander, unterschiedliche Funktionen übernehmen,

Ein "Membranfärbekomplex" der vorliegenden Erfindung ist ein Komplex, der eine Zielfindungseinheit und eine färbende Einheit umfasst und dann, wenn er mit Zielgewebe in Kontakt kommt, dort wechselwirkt und entweder direkt oder nach Bestrahlung mit Licht geeigneter Wellenlänge für das Auge sichtbar den Zielbereich färbt.

Der Ausdruck "Chromophor" bezeichnet ein Molekül, ein Element, eine Einheit oder eine Gruppe, die eine Farbe erzeugen kann, entweder direkt oder durch Bestrahlung mit Licht geeigneter Wellenlänge. Die erzeugte Farbe ist eine solche, die für das Auge sichtbar ist und somit in einem Bereich von 380 bis 780 nm liegt. Die Begriffe "physiologisch annehmbar" bzw. "ophthalmologisch annehmbar" beziehen sich auf Stoffe, Substanzen, Materialien etc., die den Organismus, dem eine Zusammensetzung, die diesen Stoff, dieses Material oder diese Substanz verabreicht wird, nicht schädigen und den dort herrschenden Zuständen angepasst sind. Der Ausdruck "ophthalmologisch annehmbar" bedeutet darüber hinaus noch, dass die entsprechende Substanz das Auge, das besonders empfindlich ist, nicht reizt oder schädigt.

Der Begriff "Trägermolekül" bezeichnet jede Art von Molekül, die dazu geeignet ist, Elemente, wie Targetingelemente oder Chromophore der vorliegenden Erfindung zu tragen. "Tragen" bedeutet in diesem Zusammenhang für ein Element jede Art der Fixierung an einem Trägermolekül, die über kovalente Bindung oder Wechselwirkungen, z.B durch Adsorption, erfolgen kann. Üblicherweise sind Elemente an ein Trägermolekül kovalent gebunden. Das Trägermolekül kann jede für die Funktion geeignete Form haben. So kann das Trägermolekül in Form von Ketten, Knäueln, Nano- oder Mikropartikeln oder sonstigen für inerte Trägermoleküle üblichen Formen vorliegen.

Der Ausdruck "Linkereinheit", wie er hier verwendet wird, bezieht sich auf ein Molekül, das zwei Gruppen miteinander verbindet, wobei die Linkereinheit im Wesentlichen inert und neutral ist, d.h. die Funktion der Elemente, die sie verbindet, nicht wesentlich beeinflusst. Eine Linkereinheit kann jedes Molekül sein, das zwei Einheiten verbindet. Ein Linkermolekül weist eine für den jeweiligen Zweck geeignete Länge auf, wobei die Einheit nicht zu lang sein sollte, um nicht die zu verbindenden Elemente zu weit voneinander zu entfernen. In der Regel trägt die Linkereinheit weder Ladung noch Funktion bei.

Ein "quaternäres Ammoniumion" ist eine Gruppe, die positiv geladen ist und einen Stickstoff und vier daran gebundene Gruppen umfasst.

Die Begriffe "wasserlöslich" und "wasserkompatibel" werden im Zusammenhang mit Polymeren bzw. dem erfindungsgemäßen Membranfärbekomplex austauschbar verwendet. Da die Übergänge zwischen Dispersion und Lösung für große Moleküle, wie solche erfindungsgemäßen Membranfärbekomplexe, die Polymere enthalten, häufig nicht festgestellt werden können, wird unter einem wasserlöslichen bzw. wasserkompatiblen Membranfärbekomplex ein solcher verstanden, der sich mit Wasser vermischen lässt und sich nicht absetzt.

Der Begriff "Zielbereich" bezieht sich auf den Bereich, in dem eine Färbung erzielt werden soll. In der Regel ist es für die vorliegende Erfindung die ILM und/oder epiretinale Membranen.

Der Begriff "wechselwirken", wenn er im Zusammenhang mit der Selektivität des Targetingelements verwendet wird, bezeichnet jede Art von Wechselwirkung zwischen Targetingelement und Membran, die dazu führt, dass das Targetingelement und damit der erfindungsgemäße Komplex an der Stelle bleibt, mit der die Wechselwirkung stattfindet. Jede Art von physikalischer Wechselwirkung kommt in betracht, solange der Komplex an dem Ort gehalten wird, wo er seine Wirkung ausüben soll.

Der Ausdruck "okulare Membranen" bezeichnet solche Membranen, die im Auge vorkommen, insbesondere epiretinale Membranen und ILM.

Der erfindungsgemäße Komplex umfasst mindestens ein Targetingelement, das den Komplex direkt zu dem Zielbereich führt, der gefärbt werden soll und in dem der Farbstoff für die gewünschte Dauer adsorbiert, gebunden oder in sonstiger Weise wechselwirken soll. Ein Targetingelement ist daher ein Element, das mit einem Zielbereich spezifisch wechselwirkt.

Es wurde nun überraschenderweise ein Strukturelement gefunden, das spezifisch mit okularen Membranen wechselwirkt. Dieses Strukturelement [A-L-Q⁺-Alk B⁻] besteht aus einer Ankergruppe, einem Linker, einer quaternären Ammoniumgruppe und einer Alkylkette mit einer Länge von 4 bis 12 Kohlenstoffatomen. Die Ankergruppe wird gebildet von einem Heteroatom, das N, O, S, P oder ein anderes zur Bindung geeignetes Heteroatom sein kann, oder einer funktionellen Gruppe, und hat die Funktion an ein Chromophor oder an ein Trägermolekül zu binden. Insbesondere kann das Heteroatom N, S oder O sein. Als funktionelle Gruppe kommen solche Gruppen inbetracht, die leicht Bindungen eingehen können, wie Alkohol-, Carbonyl-, Carboxylreste etc.

Die Ankergruppe ist über einen Linker mit einer quaternären Ammoniumgruppe verbunden. Das Linkermolekül ist ein Molekül, das die Ankergruppe mit der quaternären Ammoniumgruppe verbindet, beide Gruppen in einem vorbestimmten Abstand hält und außerdem Flexibilität beiträgt. Jedes Molekül, das eine Kettenlänge im Bereich von 1 bis 10 Atomen, bevorzugt 1 bis 8, insbesondere 1 bis 6 Atomen beiträgt und die beschriebene Funktion erfüllt, ist hier geeignet. Als besonders geeignet hat sich eine Alkylkette mit 1 bis 6, bevorzugt 2 bis 4 Kohlenstoffatomen erwiesen.

Die quaternäre Ammoniumgruppe Q⁺, die auf der einen Seite über den Linker an die Ankergruppe gebunden ist und auf der anderen Seite eine Alkylkette trägt, weist noch zwei weitere Substituenten auf. Diese beiden Substituenten sind unkritisch, solange sie für die Funktion, das Molekül zu einem Zielbereich zu führen, neutral sind, d.h. die gewünschte Funktion nicht stören. Geeignet sind beispielsweise C₁-C₄-Alkylgruppen. Bevorzugt sind die beiden Substituenten unabhängig voneinander H oder Methyl.

Weiterhin ist ein Gegenion B⁻ für die quaternäre Ammoniumgruppe vorhanden. Das Gegenion ist an sich unkritisch. Es kann daher jedes physiologisch bzw. ophthalmologisch geeignete Gegenion verwendet werden. Bevorzugt wird ein Halogenidion, z.B. Chloridion oder Bromidion, oder Hydrogenphosphation oder Hydrogensulfation als Gegenion eingesetzt.

Der dritte Teil des erfindungsgemäßen Targetingelements ist eine Alkylkette mit einer Kettenlänge von 4 bis 12 Kohlenstoffatomen, bevorzugt 6 bis 10 Kohlenstoffatomen. Es wurde gefunden, dass eine Alkylkette, die an die quaternäre Ammoniumgruppe gebunden ist, zu der Wechselwirkung mit der ILM beiträgt. Wenn die Alkylkette weniger als 4 Kohlenstoffatome aufweist, wird die selektive Bindung nicht mehr erzielt. Wenn die Kettenlänge 12 Kohlenstoffatome übersteigt, gibt es Probleme mit der Wasserlöslichkeit des erfindungsgemäßen Komplexes. Weiterhin wurde gefunden, dass es nicht von Vorteil ist, wenn die Alkylkette ungesättigte Bindungen enthält, da dadurch die Flexibilität der Kette eingeschränkt wird. Geeignet sind auch verzweigte Alkylketten, solange die Verzweigungen die Flexibilität nicht beeinträchtigen. Falls eine verzweigte Alkylgruppe verwendet wird, ist ein kritischer Faktor die Länge der längsten linearen Kette, die hier als Kettenlänge bezeichnet wird. In der Regel weist die Kette als Verzweigungen Methylgruppen auf.

Das Targetingelement kann über die Ankergruppe entweder an ein Chromophor oder an ein Trägermolekül oder an beide gebunden sein und trägt als spezifisch bindendes Element, das wiederum über einen Linker gebunden ist, ein quaternäres Ammoniumion mit einer flexiblen Alkylkette, die für die Wechselwirkung mit okularen Membranen wesentlich sind.

Überraschenderweise wurde gefunden, dass gerade die Kombination aus quaternärem Ammoniumion und flexibler Alkylkette dazu führt, dass der Komplex spezifisch mit okularen Membranen wechselwirkt. Ohne an eine Theorie gebunden zu sein, wird davon ausgegangen, dass die positive Ladung des quaternären Ammoniumions zusammen mit der hydrophoben Alkylkette dazu führt, dass das Molekül zu den okularen Membranen gezogen wird und dort aufgrund der Ladungsverteilung und des Hydrophobiegrades haften bleibt. Das Targetingelement sollte daher an ein Chromophor und/oder ein Trägermolekül in solcher Weise wechselwirkt, dass quaternäre Gruppe und hydrophober Anteil für eine Wechselwirkung mit dem Zielbereich zugänglich sind. Weitere Vorteile dieser Wechselwirkung sind, dass das Färbemittel dorthin geht, wo die Färbung erwünscht ist - an den okularen Membranen, und dort verbleibt, sodass die Menge an Färbemittel gegenüber nicht spezifisch haftenden Farbstoffen reduziert werden kann, dass außerhalb des Zielbereichs die Sicht weniger durch unspezifisch aufliegendes Färbemittel getrübt wird, und dass ein großer Teil des Färbemittels durch das Abziehen der Membran mit der okularen Membran zusammen entfernt wird.

Der zweite wesentliche Bestandteil des erfindungsgemäßen Membranfärbekomplexes ist mindestens ein Chromophor. Da der Färbekomplex zum Anfärben einer ophthalmischen Membran vorgesehen ist, muss der Farbstoff bzw. das Chromophor die oben genannten Voraussetzungen erfüllen, d.h. er darf weder reizend noch toxisch sein, muss eine ausreichende Färbung erzielen und einen wasserlöslichen oder wasserkompatiblen Färbekomplex bilden.

Als Chromophor können an sich bekannte Farbstoffe verwendet werden, solange sie die obengenannten Voraussetzungen erfüllen. Für die Anfärbung von Membranen im Auge sind insbesondere solche Farbstoffe geeignet, die einen Farbton im Bereich von blau bis grün haben, um sich gegenüber dem rötlichen Hintergrund möglichst stark abzuheben. Außerdem sollte der Farbstoff eine möglichst starke Färbung beitragen, da die Konzentration des Farbstoffs umso geringer sein kann, je stärker die Färbung ist. Eine möglichst geringe Konzentration ist erwünscht, um eine möglichst geringe Dosis anwenden zu müssen, wodurch auch bessere Verträglichkeiten erreicht werden.

Der erfindungsgemäße Komplex trägt außerdem dazu bei, dass die Dosis geringer sein muss als bei anderen bekannten Farbstoffen. Durch die gezielte Anfärbung der gewünschten Membran und durch das Anhaften aufgrund des Targetingelements kann die Menge an Farbstoff reduziert werden. Insbesondere wird der Farbstoff gezielt an den gewünschten Ort gebracht und verweilt dort.

Es wurde gefunden, dass für die erfindungsgemäße Verwendung und als Teil des erfindungsgemäßen Komplexes die Gruppe der Anthrachinone gut geeignet ist. Anthrachinonfarbstoffe sind an sich gut bekannt. Die Färbung lässt sich über eine Derivatisierung einstellen, sodass ein gewünschter Farbton durch entsprechende Derivatisierung erzielt werden kann. Außerdem können weitere Elemente wie Targetingelemente, Brückenelemente oder Trägermoleküle eingeführt bzw. gebunden werden.

Farbstoffe mit Absorption im gesamten sichtbaren Bereich können synthetisiert werden, indem die Art des Substituenten und dessen Position am Anthrachinongrundgerüst variiert werden. Insbesondere Substitutionen in den Positionen 1, 4, 5 und 8, d.h. in α-Position des Anthrachinons, haben einen starken Einfluss auf die Absorption des Farbstoffs. Unsubstituiertes Anthrachinon hat ein Absorptionsmaximum von 405 nm. Elektronendonoren bewirken eine bathochrome Verschiebung, d.h. eine Verschiebung in den längerwelligen Bereich. So hat beispielsweise 1-Aminoanthrachinon ein Absorptionsmaximum von λₘₐₓ = 475 nm, das Absorptionsmaximum von 1,4-Diaminoanthrachinon verschiebt sich um 115 nm in den längerwelligen Bereich. Gleichzeitig wird der Extinktionskoeffizient erhöht, z.B. beim 1,4-Diaminoanthrachinon um das Dreifache. Substituenten an den Positionen 2, 3, 6 und 7, d.h. einer sogenannten β-Position, haben einen geringeren Einfluss auf Farbgebung und Reaktivität. Diese Positionen können ggf. für die Anbindung von anderen Elementen genutzt werden. Da bereits durch die Einführung einer funktionellen Gruppe der Farbton verändert wird, ist es auch möglich eine der weiteren α-Positionen für die Bindung weiterer Elemente zu verwenden, ohne die Möglichkeiten der Färbung einzuschränken.

Weitere Vorteile der Anthrachinonfarbstoffe sind ihre hohe Stabilität gegenüber thermischen Einflüssen, Chemikalien und Licht.

Die α-Positionen des Anthrachinons können somit gezielt derivatisiert werden, um den gewünschten Farbton einzustellen. Weiterhin bietet das Anthrachinongerüst die Möglichkeit, ein Targetingelement einzuführen, da ausreichend Positionen für eine Derivatisierung verfügbar sind. Die Derivatisierung an sich kann mit gängigen Methoden in relativ einfacher Weise erfolgen, wie in den Beispielen beschrieben.

Wie oben ausgeführt, ist es vorteilhaft, Farbstoffe mit einer Färbung im Bereich von blau bis grün bereitzustellen. Es wurde gefunden, dass Anthrachinone, die an den zwei α-Positionen eines der beiden Phenylringe mit Aminen substituiert sind, eine blaue Farbe liefern, während Anthrachinone, bei denen an den α-Positionen eines Phenylrings eine Aminogruppe und eine Halogengruppe gebunden sind, eher im rötlichen Bereich färben. Wird ein anderes Heteroatom gebunden, z.B. O oder S verändert sich die Farbe ebenfalls. In diesen Fällen kann der zweite Phenylring unsubstituiert sein. Für eine Ausführungsform der vorliegenden Erfindung werden daher Anthrachinone, die an zwei α-Positionen mit substitutierten Aminogruppen substituiert sind, als ein blaue Farbe beitragendes Chromophor verwendet.

Insbesondere wird als Chromophor eine Verbindung der Formel I 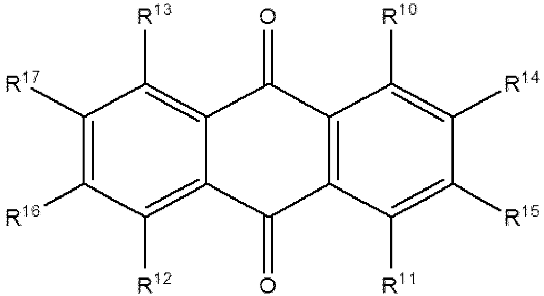 verwendet, wobei jeder der Reste R¹⁰, R¹¹, R¹² und R¹³ jeweils unabhängig voneinander Wasserstoff, C₁-C₄-Alkylreste, eine Ankergruppe, wie in Anspruch 1 definiert, oder ein Linkermolekül ist,
jeder der Reste R¹⁴, R¹⁵, R¹⁶ und R¹⁷ jeweils unabhängig voneinander Wasserstoff, ein C₁-C₄-Alkylrest oder eine Linkereinheit ist.

In einer Ausführungsform werden Anthrachinone bereitgestellt, die nicht nur den bevorzugten blauen Farbton aufweisen, sondern auch Targetingelemente tragen, mit der Eigenschaft der selektiven Bindung an okulare Membranen, ILM und ERM, indem die zur Erzielung der blauen Farbe eingeführten beiden Aminogruppen des Anthrachinons gleichzeitig als Ankergruppen des Targetingelements genutzt werden. Die Targetingelemente stören nicht die Färbung und die Anthrachinongruppe stört nicht die spezifische Bindung. In einer bevorzugten Ausführungsform trägt daher das Anthrachinon an zwei α-Positionen eine Aminogruppe, die mit einer C₁-C₆-Alkylgruppe substituiert ist, wobei das andere nicht am Anthrachinonaminostickstoff gebundene Ende der Alkylgruppe ein quaternäres Ammoniumion trägt, an das wiederum eine Alkylkette gebunden ist. Diese Kombination aus Anthrachinonfarbstoff und Targetingelement trägt sowohl die blaue Färbung als auch zur Wechselwirkung an okularen Membranen bei. Damit wird ein Membranfärbekomplex bereitgestellt, der viele vorteilhafte Eigenschaften aufweist.

Das Anthrachinon kann an ein bis vier α-Positionen mit Heteroatomgruppen, insbesondere Aminogruppen substituiert sein. Wie oben ausgeführt, kann die Anzahl der Substituenten dazu verwendet werden, den Farbton entsprechend zu beeinflussen. Wenn zwei Substituenten verwendet werden, so können diese entweder beide an den α-Positionen einer Phenylgruppe gebunden sein oder an jeder der beiden Phenylgruppen kann jeweils eine Aminogruppe gebunden sein.

In einer bevorzugten Ausführungsform ist somit die Aminogruppe so substituiert, dass sie gleichzeitig das Targetingelement des Komplexes darstellt.

In einer anderen Ausführungsform wird ein Anthrachinon, das durch geeignete Substituierung den gewünschten Farbton hat, weiter derivatisiert, indem an einer der noch freien Positionen ein Targetingelement eingeführt wird. In einer weiteren Ausführungsform tragen daher 1, 2, 3 oder 4 der α-Positionen des Anthrachinons solche Substituenten, die den Farbton beeinflussen, z.B. Elektronendonoren, wie Alkylaminogruppen, und eine der verbliebenen Positionen, z.B. auch eine β-Position, trägt, gebunden über die Ankergruppe das Targetingelement.

Diese erfindungsgemäß bevorzugten Anthrachinone sind wasserlöslich oder wasserkompatibel und können daher in dieser Form zur Anfärbung okularer Membranen verwendet werden.

Es wurde nun gefunden, dass abhängig von den Substituenten Anthrachinone in tiefere Bereiche von Zellen, z,B. in der Nachbarschaft der okularen Membranen, eindringen können. Es ist generell unerwünscht, dass zur Anfärbung oder Behandlung verwendete Verbindungen tiefer in die Zelle, z.B. in den Zellkern eindringen können, und zytotoxisch wirken können. Außerdem ist ein Anfärben von Zellen, die nicht entfernt werden und daher im Körper verbleiben, unerwünscht.

Um zu verhindern, dass ein erfindungsgemäßer Membranfärbekomplex in die Zelle eindringen kann, enthält in einer Ausführungsform der Membranfärbekomplex zusätzlich zu dem mindestens einen Targetingelement und dem mindestens einen Chromophor noch ein Trägermolekül. Das Trägermolekül wird so ausgewählt, dass es ein tieferes Eindringen in Zellen verhindert. Gleichzeitig muss das Trägermolekül wasserkompatibel sein, darf die spezifische Bindefähigkeit des Targetingelements nicht beeinträchtigen und darf weder reizend noch für Zellen schädlich sein. Mit anderen Worten, handelt es sich um ein physiologisch bzw. ophthalmologisch annehmbares Polymer. Derartige Polymere sind an sich bekannt.

Geeignet als Trägermoleküle sind solche Polymere, die funktionelle Gruppen tragen, über die Chromophore und ggf. auch Targetingelemente gebunden werden können.

Jedes Trägermolekül ist geeignet, das in dem gebildeten Färbekomplex wasserkompatibel ist, weder die Targetingfunktion noch die Färbung stört und Chromophore und/oder Targetingelelemente binden kann. Geeignet sind wasserkompatible Polymere wie z.B. von Vinylverbindungen abgeleitete Polymer und Copolymere, von Polyalkoholen abgeleitete Polymere und Copolymere, acrylbasierte Polymere und Copolymere, sowie natürliche und derivatisierte Polymere. Als Beispiele für von Vinylverbindungen abgeleitete Polymere und Copolymere können Polyvinylamin, Polyvinylalkohol, Polyvinylpyrrolidon, und die entsprechenden Copolymere genannt werden. Beispiele für von Polyalkoholen abgeleitete Polymere und Copolymere sind Polyglycerole, Polysaccharide, Polydextrine, Cyclodextrine, Polyethylenglycole, Cellulosen und Cellulosederivate, wie Hydroxypropylmethylcellulose, Hdroxyethylcellulose, Hyaluronsäure und Hyaluronsäurederivate. Als acrylbasierte Polymere können Poly-N-(2-Hydroxypropyl)methacrylamide und N-(2-Hydroxypropyl)methacrylamide genannt werden. Weitere geeignete Polymere sind Polyglutamate, auf Lactid und/oder Glykolid basierende Polymere, z.B. Polylactide auch als Cpolymer, Polyglutamate, Polyamidoamine und andere Polymere, die die oben genannten Bedingungen erfüllen. Auch Mischungen der genannten Polymere können verwendet werden.

Die Größe des Trägermoleküls kann in einem weiten Bereich variieren und ist abhängig von dem jeweils ausgewählten Polymer, der Beladung, usw. Polymere mit einem Molekulargewicht im Bereich von 900 bis zu 10 Millionen kommen inbetracht. Geeignet sind Trägermoleküle mit einem Molekulargewicht in einem Bereich von 5.000 bis 500.000 oder 1 Million, z.B. bevorzugt 20.000 bis 200.000, insbesondere 30.000 bis 100.000.

In einer Ausführungsform können Chromophore und/oder Targetingelemente an Polymere mit reaktiven Gruppen gebunden werden. Geeignet hierfür sind z.B. von Vinylaminen abgeleitete Homo- oder Copolymere, an deren Amingruppen in geeigneter Weise substituierte Anthrachinone angreifen und kovalent gebunden werden können. Für diese Ausführungsform besonders geeignet sind halogensubstituierte Anthrachinone, d.h. Anthrachinone, die an der für die Bindung vorgesehene Stelle eine Halogengruppe tragen. Die Halogengruppe sollte daher an der Position sein, an der die Bindung an das Polymer gewünscht ist.

Vorteil bei Verwendung von Anthrachinonfarbstoffen ist es, dass an beiden Phenylringen reaktive und weniger reaktive Positionen für Bindungen verfügbar sind und dass Positionen mit Einfluss auf die Farbe (α) und Positionen ohne merklichen Einfluss auf den Farbe des entstehenden Moleküls (ß) zur Verfügung stehen.

In einer Ausführungsform wird daher ein Anthrachinonderivat verwendet, das an mindestens einer α-Position ein Targetingelement aufweist, und ggf. an einer ß-Position eine Bindungsstelle für ein Trägermolekül aufweist, z.B. ein Halogen, wie Fluor oder Brom.

Der erfindungsgemäße Membranfärbekomplex kann dann in einer Ausführungsform an mindestens einer α-Position eine chromogene und zielführende Gruppe tragen und an einer ß-Position über eine Bindungsgruppe an ein Trägermolekül gebunden sein. In einer bevorzugten Ausführungsform trägt das Anthrachinonderivat ein oder zwei Targetingelemente an einem Phenylring und ist an einer ß-Position des anderen Phenylrings an ein Trägermolekül gebunden.

Die Funktion des Trägermoleküls ist es unter anderem ein Eindringen des Färbekomplexes in Zellen zu verhindern. Aus diesem Grund sollte das Trägerelement eine solche Größe haben, dass der Komplex nicht in eine Zelle aufgenommen wird. Andererseits darf das Polymer nicht so groß sein, dass es unlöslich wird bzw. nicht mehr in gelöster oder in Wasser dispergierter Form vorliegt. Das Trägermolekül kann jede geeignete Form haben. Es kann sich um eine Polymerkette handeln, an der in beliebiger Reihenfolge Chromophore und Targetingelemente gebunden sind. Es kann sich auch um verzweigte Polymere handeln, an deren Zweigen Chromophore und/oder Targetingelemente gebunden sind. In einer weiteren Ausführungsform kann das Polymer in Form von Kügelchen vorliegen, an deren Oberfläche Chromophore und/oder Targetingelemente gebunden sind.

Da das Targetingelement aufgrund seiner Struktur auch an dem Chromophor gebunden sein kann, ist in einer anderen Ausführungsform der Komplex so aufgebaut, dass an dem Trägermolekül Chromophoreinheiten gebunden sind, an denen wiederum Targetingelemente gebunden sind.

Die Anzahl der an einem Trägermolekül gebundenen Chromophore und/oder Targetingelemente hängt ab von der Größe des Trägermoleküls, der Art der Monomereinheiten, der Bindefähigkeit des Targetingelements, der Färbefähigkeit des oder der Chromophore. Der Fachmann kann mit einfachen Routineversuchen das jeweils optimale Verhältnis von Trägermolkül/Chromophor/Targetingelement herausfinden. Das Verhältnis von Chromophoreinheiten zu Targetingelementen ist ebenfalls unkritisch und richtet sich z.B. nach Farbstärke des Chromophors, Affinität des Targetingelements, gewünschtem Farbton. Es wurde gefunden, dass ein Überschuss an Targetingelementen im Vergleich zu Chromophoren oft vorteilhaft ist. Ein molares Verhältnis von Chormophoreinheiten zu Targetingelementen im Bereich von 1:20 bis 5:1, insbesondere 1:10 bis 2:1 wird als geeignet angesehen.

Üblicherweise wird für den erfindungsgemäßen Membranfärbekomplex nur eine Art von Chromophor verwendet. Möglich ist allerdings auch, an ein Trägermolekül verschiedene Chromophore zu binden oder eine Mischung von Trägermolekülen, die jeweils unterschiedliche Chromophore tragen, zu verwenden. Wesentlich ist dabei nur, den gewünschten Farbton zu erzielen, um dann okulare Membranen anzufärben.

Der Aufbau des erfindungsgemäßen Membranfärbekomplexes ermöglicht es auch, ein Färbemittel zur Verfügung zu stellen, das in unterschiedlichen Farben erscheint, je nach verwendeter Lichtquelle. So kann beispielsweise eine Kombination von erfindungsgemäßen Membranfärbekomplexen mit unterschiedlichen Chromophoren verwendet werden, wobei ein Chromophor bei einer Wellenlänge färbt und ein anderes Chromophor bei einer anderen Wellenlänge des eingestrahlten Lichts sichtbar wird.

Es ist auch möglich, als Chromophor eine Verbindung mit fluoreszierenden Eigenschaften zu verwenden.

Der erfindungsgemäße Membranfärbekomplex weist ein Targetingelement auf, das selektiv mit okularen Membranen wechselwirkt. Es ist somit möglich, okulare Membranen als Zielbereich gezielt anzufärben. Ein weiterer Gegenstand der Erfindung ist daher die Verwendung des erfindungsgemäßen Membranfärbekomplexes zur Anfärbung von okularen Membranen.

Die Zeichnungen dienen der Erläuterung der Erfindung, wobei
Figur 1 ein Diagramm zeigt mit der graphischen Auftragung der UV/Vis-spektroskopischen Messergebnisse der BBG-Referenz (blau) und BBG-MS-Lösung (rot) in Konzentrationen von 0,005 bis 0,08 mg/mL.
Figur 2 ein Diagramm zeigt mit der graphischen Auftragung der UV/Vis-spektroskopischen Messergebnisse der 7a- und b-Referenzen (blau und rot) und 7a- und b-MS-Lösung (lila und grün) in Konzentrationen von 0,005 bis 0,16 mg/mL.
Figur 3 ein Diagramm zeigt mit der graphischen Auftragung der UV/Vis-spektroskopischen Messergebnisse der 7c- und d-Referenzen (blau und rot) und 7c- und d-MS-Lösung (lila und grün) in Konzentrationen von 0,005 bis 0,16 mg/mL.
Figur 4 ein Diagramm zeigt mit der graphischen Auftragung der UV/Vis-spektroskopischen Messergebnisse der ICG-Referenz (blau) und ICG-MS-Lösung (rot) in Konzentrationen von 0,005 bis 0,04 mg/mL.

### BEISPIELE

Die Erfindung wird anhand der nachfolgenden Beispiele weiter erläutert, ohne sie dadurch zu beschränken. Alle Konzentrationsangaben in den Beispielen beziehen sich auf mg Substrat pro ml Gesamtlösung. Wenn nicht anders angegeben wurden alle Reaktionen bei Raumtemperatur, d.h. etwa 25°C durchgeführt.

Für die Analytik wurden folgende Verfahren und Bedingungen verwendet:

### ¹H-Kernresonanz-Spektroskopie (¹H-NMR-Spektroskopie)

300 MHz und 600 MHz NMR-Spektren wurden mit einem FT-NMR-Spektrometer vom Typ Bruker Avance III bei Raumtemperatur aufgenommen. Die Angaben der chemischen Verschiebung beziehen sich auf das deuterierte Lösemittelsignal als internen Standard. Spinnmultiplizitäten wurden mit s (Singulett), d (Dublett), dd (Dublett von Dublett), t (Triplett) oder m (Multiplett) abgekürzt.

### ¹³C-Kernresonanz-Spektroskopie (¹³C-NMR-Spektroskopie)

Die Messungen erfolgten ebenfalls an einem FT-NMR-Spektrometer vom Typ Bruker Avance III bei 75 MHz und 150 MHz bei Raumtemperatur. Die Angaben der chemischen Verschiebung beziehen sich auf das deuterierte Lösemittelsignal als internen Standard.

### MALDI-TOF-MS

Die Messungen wurden mit einem Bruker Ultraflex TOF (time off light) Massenspektrometer aufgenommen. Das Gerät arbeitet mit einem 337 nm Stickstoff-Laser, sowohl im Linearmodus als auch im Reflektormodus. Die Proben wurden in einem geeigneten Lösemittel gelöst und unter Verwendung von 2-(4-Hydroxyphenylazo)benzoesäure (HABA) versetzt.

### ESI-MS

Die Messungen wurden mit einem Massenspektrometer Typ Ion-Trap-API Finnigan LCQ Deca aufgenommen. Die zu bestimmenden Substanzen wurden in einem geeigneten Lösemittel mit einer Konzentration von 1 mg/mL gelöst. Die Ionisierung erfolgte per Elektrospray-Ionisation.

### LC-MS

Die Messungen wurden mit einem Gerät Typ Agilent Technologies 6120 mit einem Absorptions-Detektor (214 nm) in Kombination mit einem Agilent Quadrupol Massenspektrometer durchgeführt. Verwendet wurde eine Säule der Firma Agilent SB-C₁₈ 1,8 µm (2,1 x 50 mm) mit einem Lösemittelgemisch von H₂O:MeCN + 0,1 % Ameisensäure bei Raumtemperatur. Die Flussrate betrug 0,4 mL/min. bei einem Lösemittelgemisch von 95:5 H₂O:MeCN und 5:95 H₂O:MeCN.

### GC-MS

Die Messungen wurden mit einem Thermo Finnigan Trace DSQ (Dual-Stage Quadrupol) aufgenommen. Die Ionisierung der Proben erfolgte durch Elektronenstoßionisation (EI).

### FT-IR-Spektroskopie

FT-IR-Spektren wurden mit Hilfe eines Nicolet 6700 FT-IR Spektrometers mit ATR-Aufsatz der Firma LOT bei Raumtemperatur aufgezeichnet. Die Kalibrierung der Wellenzahlenskala erfolgte mit einem HeNe-Laser. Die Messungen wurden in einem Bereich von 4000-300 cm⁻¹ aufgenommen.

### UV/Vis-Spektroskopie

Für UV/Vis-spektroskopische Aufnahmen wurde ein zweistrahliges Spektrometer Specord® 210 Plus der Analytik Jena AG verwendet. Die Messungen wurden bei Raumtemperatur zwischen 300 und 800 nm unter Verwendung einer Quarzglasküvette der Schichtdicke 1 cm durchgeführt. Die Auswertung erfolgte mithilfe der Software WinAspect Plus der Firma Analytik Jena AG.

### Dünnschichtchromatographie

Für die Dünnschichtchromatographie wurden Platten der Firma Merck verwendet (Kieselgel 60 F₂₅₄ auf Aluminiumfolie).

### HPLC

RP-HPLC-Messungen (*reversed phase high prerformance liquid chromatography*) wurden mit Hilfe eines Agilent 1200 mit Absorptions- und Fluoreszenz-Detektor aufgenommen. Verwendet wurde eine Agilent Zorbax Eclipse XDB-C₁₈ Säule (4,6 x 100 mm) mit einer Flussrate von 1 mL/min. bei 60°C. Eingesetzt wurde ein binäres Eluiermittelgemisch aus H₂O und MeCN mit 0,1 % Trifluoressigsäure. Die Messungen wurden mittels Gradientenelution über einen Zeitraum von 10 min. von Acetonitril:Wasser 5:95 nach 95:5 durchgeführt.

### Beispiel 1

### Synthese von 1,4-Difluoranthrachinon (3)

13,4 g (0,9 mol) Phthalsäureanhydrid (1), 48,8 g (0,37 mol) AlCl₃ und 108 mL (2 mol) 1,4-Difluorbenzol (**2**) werden für 48 h unter Rückfluss erhitzt. Das überschüssige 1,4-Difluorbenzol wird anschließend mittels Destillation zurückgewonnen. Dem braunen Rückstand werden 400 mL 1N Salzsäure hinzugegeben und mit drei Mal je 600 mL Chloroform extrahiert. Die vereinten organischen Phasen werden unter vermindertem Druck auf ca. 50 mL eingeengt. Das Zwischenprodukt wird anschließend mit mindestens 100 mL niedrigsiedendem Petrolether ausgefällt. Der bräunliche Feststoff wird abfiltriert und anschließend getrocknet. (Ausbeute: 66,7 % d. Th.) Der Feststoff wird in 200 g Polyphosphorsäure vorgelegt und für 2 h auf 140 °C erhitzt. Das zähflüssige Reaktionsgemisch wird nach Abkühlen auf Raumtemperatur in 1 L Eiswasser gegeben und mit drei Mal mit je 400 mL Dichlormethan extrahiert. Nach Einengen der organischen Phase unter vermindertem Druck und säulenchromatographischer Aufreinung über Kieselgel (Hexan:Essigester [7:1]) kann das Produkt **3** isoliert werden. 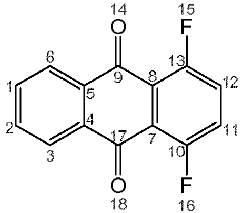
**Ausbeute:** 13,2 g (54,1 mmol) 60,1% d. Th. (Lit.= 50 %)
**FT-IR (Diamant):** v= 3084 (s, υ_{C=C}), 1678 (s, υ_{C=O}), 1587 (m, υ_{C=C}), 1249 (vs, υ_{C-F}), 719 (s, δ_{Ar-H}) *cm⁻¹*.
**¹H-NMR (300 MHz, CDCl₃, RT):** δ= 8,20-8,29 (m, 2H, Ar-**H^{3,6}**), 7,76-7,85 (m, 2H, Ar-**H^{1,2})**, 7,43-7,53 (m, 2H, Ar-**H^{11,12})** *ppm.*
**¹³C-NMR (300 MHz, DMSO-d₆, RT):** δ= 180,23 (C^{9,17}), (158,55, 155,10) (C^{10,13}), 134,40 (C^{1,2}), 133,04 (C^{4,5}), 126,23 (C^{3,6}), 124,66-125,25 (C^{7,8}), 121,43-121,60 (C^{11,12}) *ppm.*
**EI-MS** m/z: 244 [M]

### Beispiel 2

### Synthese von 1,4-Bis[[2-(dimethylamino)propyl]amino]anthrachinon (5)

3,0 g (12,3 mmol) 1,4-Difluoranthrachinon (**3**) werden mit 3,1 mL (24,6 mmol) *N,N-*Dimethylaminopropylamin (**4**) in 25 mL DMSO unter Stickstoffatmosphäre auf ca. 100 °C erhitzt. Nach 24 h Rühren wird das Reaktionsgemisch auf Raumtemperatur abgekühlt und mit Wasser verdünnt. Das blaue Rohprodukt wird mit CHCl₃ extrahiert, unter vermindertem Druck eingeengt und säulenchromatographisch über Kieselgel aufgereinigt (CH₂Cl₂:MeOH:NEt₃ [3:4:0,1]). 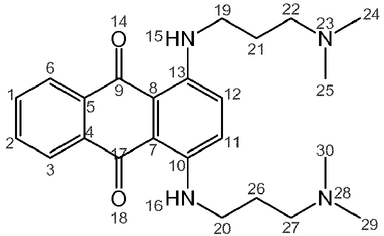
**Ausbeute:** 2,1 g (5,2 mmol) 42,3 % d. Th.
**FT-IR (Diamant):** v= 3425 (w, υ_{NH}), 3068 (w, υ_{Ar-H}), 2948 (m, υ_{CH2}), 2859 (m, υ_{CH2}), 2787 (m, υ_{(N-CH3)C-H}), 2766 (m, υ(_{N-CH3)N-C}), 1642 (w, υ_{C=O}), 1591 (m, υ_{C=C}), 1567 (m, υ_{C=C}), 1556 (s, υ_{C=C}), 1457 (m, δ_{CH2,CH3}), 731 (s, υ_{Ar-H}) *cm⁻¹*.
**¹H-NMR (300 MHz, CDCl₃, RT):** δ= 10,83 (t, ³J_{H,H}=5,35 Hz, 2H, N**H**^{15,16}), 8,34 (dd, ³J= 5,92, ⁴J= 3,27 Hz, 2H, Ar-**H**^{3,6}), 7,69 (dd, ³J= 5,91, ⁴J= 3,31 Hz, 2H, Ar-**H**^{1,2}), 7,30 (s, 2H, Ar-**H^{11,12}**), 3,48 (m, 4H, C**H₂**^{19,20}), 2,45 (t, ³J=6,94 Hz, 4H, C**H₂**^{22,27}), 2,27 (s, 12H, N**Me₂**^{24,24,29,30}), 1,92 (t, ³J= 7,1 Hz, 4H, C**H₂**^{21,26}) *ppm.*
**¹³C-NMR (75 MHz, DMSO-d**₆**, RT):** δ= 182,41 (C^{9,17}), 146,42 (C^{10,13}), 134,70 (C^{1,2}), 132,07 (C^{4,5}), 126,12 (C^{3,6}), 123,80 (C^{7,8}), 109,85 (C^{11,12)}, 57,78 (C^{22,27}), 45,67 (C^{24,25,29,30}), 41,03 (C^{19,20}), 28,05 (C^{21,26}) *ppm.*
**ESI-MS** *m*/*z:* 205,3 [M+2H]²⁺
**Elementaranalyse:** theoretische Werte: C: 70,56; H: 7,90; N: 13,71 Analysenergebnisse: C: 70,65; H: 7,70; N: 13,58

### Beispiel 3

### Synthese von N,N'-(((9,10-Dioxo-9,10-dihydroanthracen-1,4-diyl)bis(azanediyl))-bis(propan-3,1-diyl))bis(N,N-dimethylbutan-1-aminium)bromid (7a)

0,5 g (1,23 mmol) 1,4-Bis[[2-(dimethylamino)propyl]amino]anthrachinon (**5**) werden mit 1,68 g (1,3 mL, 12,25 mmol) 1-Brombutan (**6a**) in ca. 5 mL Aceton für 24 h zum Rückfluss erhitzt. Nach Abkühlen der Lösung auf Raumtemperatur wird das Produkt in Hexan vollständig ausgefällt, abfiltriert und mit Hexan gewaschen. Der blaue Feststoff wird im Hochvakuum getrocknet. 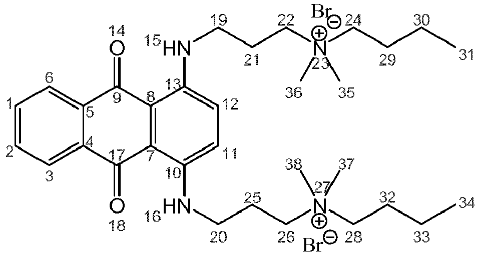
**Ausbeute:** 0,81 g (1,19 mmol) 97 % d. Th.
**FT-IR (Diamant):** v= 3404 (br, υ_{NH}), 3006 (w, υ_{Ar-H}), 2958 (m, υ_{CH2}), 2871 (m, υ_{CH2}), 1641 (w, υ_{C=O}), 1607 (w, υ_{C=C}), 1594 (m, υ_{C=C}), 1572 (s, υ_{C=C}), 1519 (m, υ_{C=C}), 1467 (m, δ_{CH2,CH3}), 724 (s, υ_{Ar-H}) *cm⁻¹.*
**¹H-NMR (300 MHz, DMSO-d₆, RT):** δ[ppm]= 10,79 (t, ³J= 5,82 Hz, 2H, N**H**^{15,16}), 8,24 (dd, ³J= 5,85 Hz, ⁴J= 3,4 Hz, 2H, Ar-**H**^{3,6}), 7,83 (m, 2H, Ar-**H**^{1,2}), 7,57 (s, 2H, Ar-**H**^{11,12}), 3,55 (m, 4H, C**H₂**^{19,20}), 3,44 (m, 4H, C**H₂**^{22,26)}, 3,31 (m, 4H, C**H₂**^{24,28}), 3,07 (s, 12H, N**Me₂**³⁵⁻³⁸), 2,09 (m, 4H, C**H₂**^{21,25}), 1,64 (m, 4H, C**H₂**^{29,32}), 1,29 (m, 4H, C**H₂**^{30,33}), 0,91 (t, ³J= 7,3 Hz 6H, C**H₃**^{31,34}) *ppm.*
**¹³C-NMR** (75 MHz, **DMSO-d₆,** RT): δ= 181,12 (C^{9,17}), 145,63 (C^{10,13)}, 133,72 (C^{1,2}), 132,55 (C^{4,5}), 125,67 (C^{3,6}), 124,52 (C^{7,8}), 108,84 (C^{11,12}), 62,89 (C^{24,28}), 60,62 (C^{22,26}), 50,24 (C^{35,36,37,38}), 39,5 (C^{19,20} unter DMSO-d₆-Signal), 23,67 (C^{29,32}), 22,73 (C^{21,25}), 19,13 (C^{30,33}), 13,40 (C^{31,34}) *ppm.*
**LC-MS** *m*/*z*: 261,2 [M-2Br]²⁺
**HP-LC:** 97,07 % 6,03 min.

### Beispiel 4

### Synthese von N,N'-(((9,10-Dioxo-9,10-dihydroanthracen-1,4-diyl)bis(azanediyl))-bis(propan-3,1-diyl))bis(N,N-dimethyloctan-1-aminium)bromid (7b)

0,65 g (1,33 mmol) 1,4-Bis[[2-(dimethylamino)propyl]amino]anthrachinon (**5**) werden mit 2,6 g (2,3 mL, 13,3 mmol) 1-Bromoctan (**6b**) in ca. 5 mL Aceton für 24 h zum Rückfluss erhitzt. Nach Abkühlen der Lösung auf Raumtemperatur wird das Produkt in Hexan vollständig ausgefällt, abfiltriert und mit Hexan gewaschen. Der blaue Feststoff wird im Hochvakuum getrocknet. 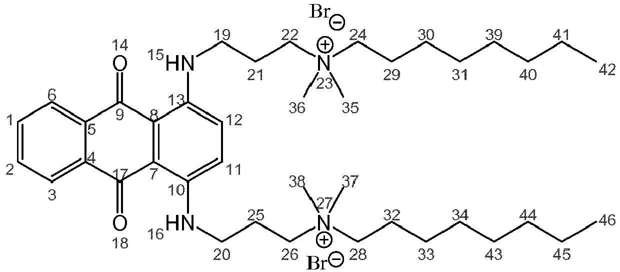
**Ausbeute:** 0,99 g (1,25 mmol) 94 % d. Th.
**FT-IR (Diamant)**: v= 3419 (br, υ_{NH}), 3004 (w, υ_{Ar-H}), 2953 (m, υ_{CH2}), 2923 (m, υ_{CH2}), 2854 (m, υ_{CH2}), 1645 (w, υ_{C=O}), 1595 (w, υ_{C=C}), 1576 (m, υ_{C=C}), 1588 (s, υ_{C=C}), 1467 (m, δ_{CH2,CH3}), 717 (s, υ_{Ar-H}) *cm⁻¹*.
**¹H-NMR (300 MHz, DMSO-d₆, RT):** δ[ppm]= 10,79 (t, ³J= 5,84 Hz, 2H, N**H**^{15,16}), 8,25 (dd, ³J= 5,90 Hz, ⁴J=3,30 Hz, 2H, Ar-**H**^{3,6}),7,82 (m, 2H, Ar-**H**^{1,2}), 7,57 (s, 2H, Ar-**H**^{11,12}), 3,55 (m, 4H, C**H₂**^{19,20}), 3,43 (m, 4H, C**H₂**^{22,26}), 3,29 (m, 4H, C**H₂**^{24,28}), 3,06 (s, 12H, N**Me₂**³⁵⁻³⁸), 2,09 (m, 4H, C**H₂**²¹⁻²⁵), 1,64 (m, 4H, C**H₂**^{29,32}), 1,12-1,31 (m, 20H, C**H₂**^{30,31,33,34,39-41,43-45}), 0,82 (m, 6H, C**H₃**^{42,46}) *ppm.*
**¹³C-NMR (75 MHz, DMSO-d₆, RT):** δ= 181,10 (C^{9,17}), 145,63 (C^{10,13}), 133,74 (C^{1,2}), 132,56 (C^{4,5}), 125,71 (C^{3,6}), 124,56 (C^{7,8}), 108,84 (C^{11,12}), 62,85 (C^{24,28}), 60,38 (C^{22,26}), 50,32 (C³⁵⁻³⁸), 39,5 (C^{19,20} unter DMSO-d₆-Signal), 31,16 (C^{40,44)}, 28,50 (C^{31,34,39,43}), 25,80 (C^{30,33}), 22,73 (C^{29,32}), 22,02 (C^{21,25}), 21,71 (C^{41,45}), 13,94 (C^{42,46}) *ppm*.
**LC-MS** m/z: 317,3 [M-2Br]²⁺
**HP-LC:** 96,98 % 7,75 min.

### Beispiel 5

### Synthese von N,N'-(((9,10-Dioxo-9,10-dihydroanthracen-1,4-diyl)bis(azanediyl))-bis(propan-3,1-diyl))bis(N,N-dimethyldecan-1-aminium)bromid (7c)

0,33 g (0,81 mmol) 1,4-Bis[[2-(dimethylamino)propyl]amino]anthrachinon (**5**) werden mit 1,69 mL (8,1 mmol) 1-Bromdecan **(6c)** in 5 mL Aceton für ca. 48 h zum Rückfluss erhitzt. Nach Abkühlen der Lösung auf Raumtemperatur wird das Produkt in Hexan vollständig ausgefällt, abfiltriert und mit Hexan gewaschen. Der blaue Feststoff wird im Hochvakuum getrocknet. 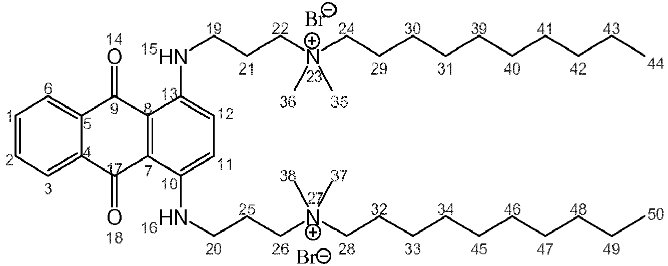 **Ausbeute:** 0,68 g (0,8 mmol) 99 % d. Th.
**FT-IR (Diamant):** v= 3419 (br, υ_{NH}), 3007 (w, υ_{Ar-H}), 2948 (m, υ_{CH2}), 2920 (m, υ_{CH2}), 2852 (m, υ_{CH2}), 1639 (w, υ_{C=O}), 1595 (m, υ_{C=C}), 1579 (m, υ_{C=C}), 1562 (s, υ_{C=C}), 1467 (m, δ_{CH2,CH3}), 731 (s, υ_{AR-H}) *cm⁻¹.*
**¹H-NMR (300 MHz, DMSO-d₆, RT):** δ= 10,79 (t, ³J= 5,73 Hz, 2H, N**H**^{15,16}), 8,24 (dd, ³J= 5,88 Hz, ⁴J= 3,31 Hz, 2H, Ar-**H**^{3,6}), 7,82 (m, 2H, Ar-**H**^{1,2}), 7,57 (s, 2H, Ar-**H**^{11,12}), 3,55 (m, 4H, C**H₂**^{19,20}), 3,43 (m, 4H, C**H₂**^{22,26}), 3,29 (m, 4H, C**H₂**^{24,28}), 3,06 (s, 12H, N**Me₂**³⁵⁻³⁸), 2,08 (m, 4H, C**H₂**^{21,25}), 1,63 (m, 4H, C**H₂**^{29,32}), 1,12-1,32 (m, 28H, C**H₂**^{30,31,33,34,39-43,45-49}), 0,83 (m, 6H, C**H₃**^{44,50}) *ppm.*
**¹³C-NMR (75 MHz, DMSO-d₆, RT):** δ= 181,17 (C^{9,17}), 145,70 (C^{10,13}), 133,79 (C^{1,2}), 132,62 (C^{4,5}), 125,77 (C^{3,6}), 124,62 (C^{7,8}), 108,90 (C^{11,12}), 62,87 (C^{24,28}), 60,41 (C^{22,26}), 50,39 (C³⁵⁻³⁸), 39,5 (C^{19,20} unter DMSO-d₆-Signal), 31,31 (C^{42,48}), 28,57-28,94 (C^{31,34,39-41,45-47}), 25,82 (C^{30,33}), 22,75 (C^{29,32}), 22,11 (C^{21,25}), 21,74 (C^{43,49}), 13,98 (C^{44,50}) *ppm*.
**LC-MS** m/z: 345,4 [M-2Br]²⁺
**HP-LC:** 96,92 % 8,57 min.

| | | | | |
|---|---|---|---|---|
| **Elementaranalyse:** | theoretische Werte: | C: 62,11; | H: 8,77; | N: 6,58 |
| | Analysenergebnisse: | C: 61,87; | H: 8,72; | N: 6,42 |

### Beispiel 6

### Synthese von N,N'-(((9,10-Dioxo-9,10-dihydroanthracen-1,4-diyl)bis(azanediyl))-bis(propan-3,1-diyl))bis(N,N-dimethyldodecan-1-aminium)bromid (7d)

0,35 g (0,86 mmol) 1,4-Bis[[2-(dimethylamino)propyl]amino]anthrachinon (**5**) werden mit 0,85 g (0,89 mL, 3,43 mmol) 1-Bromdodecan (**6d**) in ca. 2 mL Aceton für 24 h zum Rückfluss erhitzt. Nach Abkühlen der Lösung auf Raumtemperatur wird das Produkt in Hexan vollständig ausgefällt, abfiltriert und mit Hexan gewaschen. Der blaue Feststoff wird im Hochvakuum getrocknet. 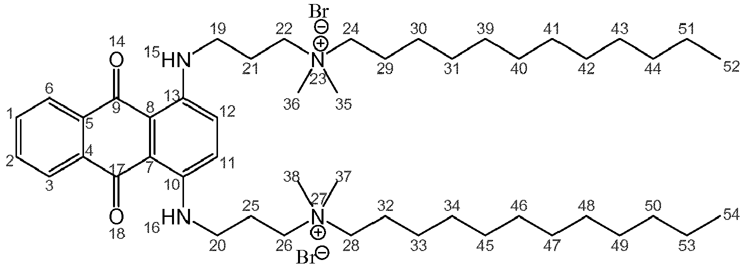
Ausbeute: 0,71 g (0,79 mmol) 91 % d. Th.
FT-IR (Diamant): v= 3425 (br, υ_{NH}), 3006 (w, υ_{Ar-H}), 2948 (m, υ_{CH2}), 2919 (s, υ_{CH2}), 2851 (m, υ_{CH2}), 1639 (w, υ_{C=O}), 1595 (m, υ_{C=C}), 1580 (m, υ_{C=C}), 1562 (s, υ_{C=C}), 1468 (m, δ_{CH2,CH3}), 731 (s, υ_{Ar-H}) *cm⁻¹*.
**¹H-NMR** (300 MHz, **DMSO-d₆,** RT): δ= 10,78 (m, 2H, N**H**^{15,16}), 8,25 (m, 2H, Ar-**H**^{3,6}), 7,82 (m, 2H, Ar-**H**^{1,2}), 7,56 (s, 2H, Ar-**H**^{11,12}), 3,55 (m, 4H, C**H₂**^{19,20}), 3,21-3,46 (m, C**H₂**^{22,26,24,28} von H₂O-Signal überlagert), 3,05 (s, 12H, N**Me₂**³⁵⁻³⁸), 2,08 (m, 4H, C**H₂**^{**2**1,25}), 1,63 (m, 4H, C**H₂**^{29,32}), 1,11-1,32 (m, 36H, C**H₂**^{30,31,33,34,39-51,53}), 0,84 (m, 6H, C**H**₃^{52,54}) *ppm.*
**¹³C-NMR (75 MHz, DMSO-d₆, RT):** δ= 181,17 (C^{9,17}), 145,65 (C^{10,13}), 133,77 (C^{1,2}), 132,61 (C^{4,5}), 125,74 (C^{3,6}), 124,54 (C^{7,8}), 108,89 (C^{11,12}), 62,80 (C^{24,28}), 60,38 (C^{22,26}), 50,38 (C³⁵⁻³⁸), 39,5 (C^{19,20} unter DMSO-d₆-Signal), 31,29 (C^{44,50}), 28,53-29,1 (C^{31,34,39-43,45-49}), 25,78 (C^{30,33}), 22,70 (C^{29,32}), 22,09 (C^{21,25}), 21,69 (C^{51,53}), 13,96 (C^{52,54}) *ppm.*
**LC-MS** m/z: 373,4 [M-2Br]²⁺
**HP-LC:** 96,44 % 9,79 min.

### Beispiel 7

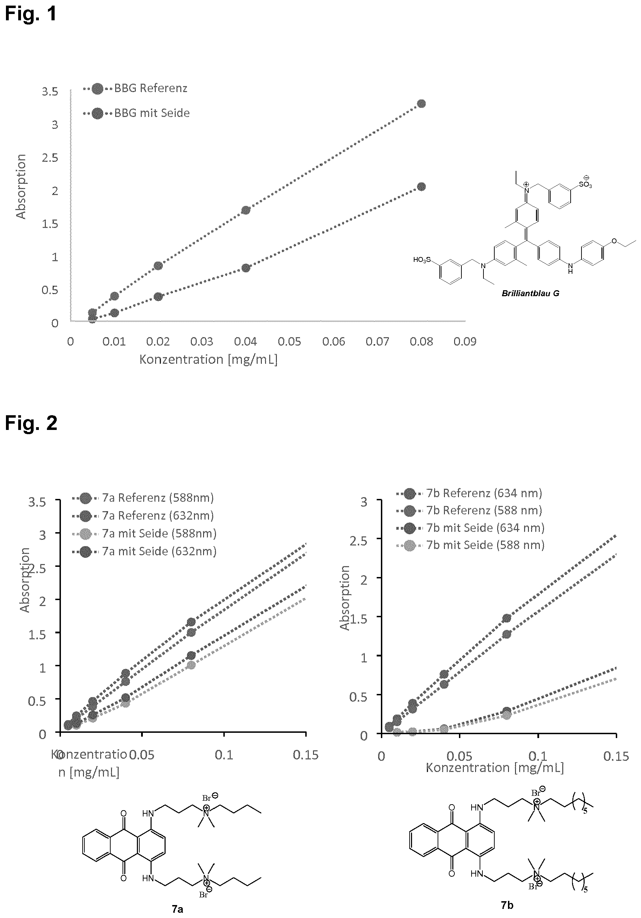

Es wurde ein ILM-Modellsubstrat, das der ILM ähnlich ist, verwendet, um die Bindefähigkeit von bekannten Farbstoffen und erfindungsgemäßen Färbekomplexen zu testen. Es wurde gefunden, dass als ILM-Modellsubstrat Seidengranulat gut geeignet ist. Zur Überprüfung der Anbindung verschiedener Farbstoffe an ILM-Modellsubstrat wurden UV/Vis-Messungen der Farbstoffe *Brilliantblau* G (BBG), Verbindungen **7a-d**, und *Indocyaningrün* (ICG) in verschiedenen Konzentrationen sowohl vor als auch nach Behandlung mit Modellsubstrat durchgeführt. Ziel dieser Messungen ist ein Vergleich der Anbindungsstärke der Farbstoffe untereinander. Die Ergebnisse sind unten aufgeführt und in den Figuren 1 bis 4 jeweils als Diagramm dargestellt.

Zur Herstellung der Farbstoff-Stammlösungen mit einer Konzentration von 5 mg/mL wurde phosphatgepufferte Salzlösung (PBS) mit physiologischem pH-Wert von 7,4 verwendet. Aufgrund der schlechten Löslichkeit von ICG in Salzlösungen wurde doppelt destilliertes Wasser benutzt. Die angesetzten Konzentrationen sind in
Tabelle gezeigt.

**Tabelle 1: Herstellung der Farbstofflösungen für die UV/Vis-kontrollierte Anbindungsstudie an ILM-Modellsubstrat**

| Konzentration [mg/mL] | Zusammensetzung Farbstofflösung |
|---|---|
| 0,005 | 6.000 µL PBS + 6 µL Stammlösung |
| 0,01 | 5.994 µL PBS + 12 µL Stammlösung |
| 0,02 | 5.982 µL PBS + 24 µL Stammlösung |
| 0,04 | 5.958 µL PBS + 48 µL Stammlösung |
| 0,08 | 5.910 µL PBS + 96 µL Stammlösung |
| 0,16 | 5.814 µL PBS + 192 µL Stammlösung |

Um eine möglichst genaue Messung zu gewährleisten, wurden die Farbstoff-Modellsubstrat-Lösungen und die Referenzen nicht getrennt hergestellt, sondern ebenfalls als Stammlösungen verwendet. Hierzu wurde das Zweieinhalbfache der benötigten Menge analog Tabelle 1 hergestellt.

Anschließend wurden 6 mg ILM-Modellsubstrat mit 6 mL Farbstofflösung, sowie eine Referenz mit ausschließlich 6 mL Farbstofflösung exakt 10 Minuten gerührt und danach gemeinsam 8 Minuten bei 6000 rpm zentrifugiert. Die Lösungen wurden fotografiert und die vorsichtig entnommenen Überstände UV/Vis-spektroskopisch analysiert. Die Absorptionsmaxima λₘₐₓ mit Modellsubstrat wurden ins Verhältnis zum Absorptionsmaximum λₘₐₓ der Referenz gesetzt. So konnten die Anbindungsstärken der Farbstoffe untereinander verglichen werden. Die graphischen Ergebnisse sind in Figur 1 gezeigt. Die tabellarische Zusammenfassung der Messergebnisse ist im Folgenden gezeigt.

### a) UV/Vis-Messungen des Vitalfarbstoffs Brilliantblau G (BBG) (Vergleich)

Wie bereits erläutert ist *Brilliantblau G* in der Chromovitrektomie ein häufig verwendeter Farbstoff mit selektiver Anfärbung der *membrana limitans interna.* Aus diesem Grund wurde auch in Anbindungsversuchen mit Modellsubstrat eine gute Färbung und demnach starke Abnahme des Absorptionsmaximums nach Behandlung mit Modellsubstrat erwartet.

Wie anhand von Figur 1 gezeigt werden kann, bestätigt sich die erwartet gute Anbindung an das Modellsubstrat. Betrachtet wurde dabei lediglich das stärkste Absorptionsmaximum, welches für die blaue Farbgebung verantwortlich ist. Das Maximum der Referenzen liegt dabei bei 585 nm. Die mit Modellsubstrat behandelten Proben weisen eine leichte Verschiebung des Maximums auf, das sich mit steigender Konzentration dem der Referenz annähert. Die Referenzen zeigen eine deutlich stärkere Absorption als die mit Modellsubstrat behandelten Proben. Dies spricht für eine gute Wechselwirkung zwischen Farbstoff und Modellsubstrat. Tabelle 2 sind die jeweiligen Absorptionsmaxima der Farbstofflösungen zu entnehmen. Das Verhältnis von λₘₐₓ mit Modellsubstrat zu λₘₐₓ der zugehörigen Referenz bestätigt die graphische Darstellung der Anbindungsstärke. Bei einer Konzentration von 0,005 mg/mL weist die mit Modellsubstrat behandelte Probe lediglich 24 % des Referenz-Maximums auf. Dies entspricht einer Anbindung des Farbstoffes von 76 %. Mit zunehmender Konzentration der Lösung nimmt die Anbindung ab. Bei einer Konzentration von 0,08 mg/mL kann lediglich eine Anbindung von 48 % des Farbstoffes nachgewiesen werden.

**Tabelle 2: UV/Vis-spektroskopische Messergebnisse der BBG-Referenz und BBG-Modellsubstrat(MS)-Lösung in Konzentrationen von 0,005 bis 0,08 mg/mL**

| Konzentration [mg/mL] | λₘₐₓ Referenz (585 nm) | λₘₐₓ mit MS (x nm) | Verhältnis λₘₐₓ mit MS und Referenz |
|---|---|---|---|
| 0,005 | 0,1241 | 0,0299 (603 nm) | 24 % |
| 0,01 | 0,3755 | 0,1217 (595 nm) | 32 % |
| 0,02 | 0,835 | 0,3672 (589 nm) | 44 % |
| 0,04 | 1,6741 | 0,8006 (587 nm) | 48 % |
| 0,08 | 3,2859 | 2,0313 (585 nm) | 62 % |

### b) UV/Vis-Messungen der Anthrachinonfarbstoffe 7a-d (erfindungsgemäß)

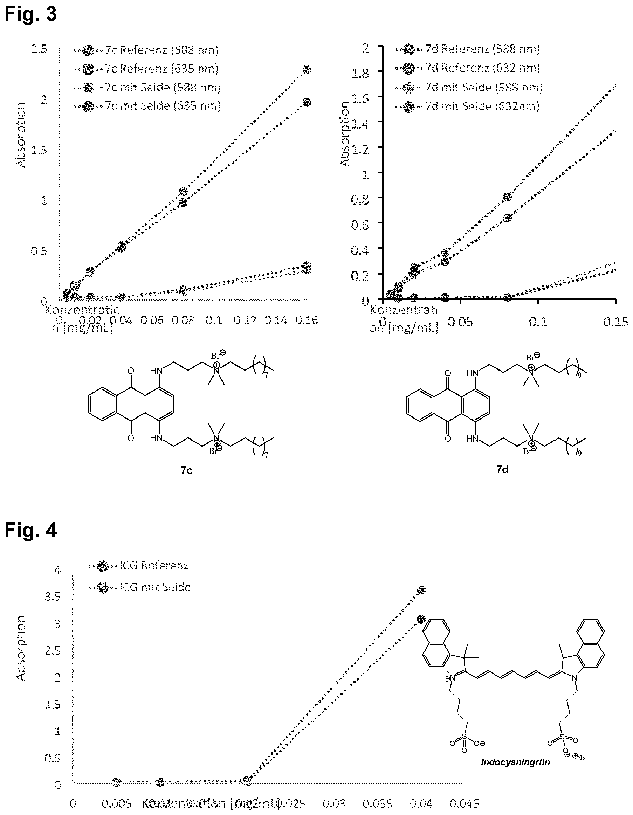

Die erfindungsgemäßen Anthrachinonfarbstoffe **7a-d** wurden ebenfalls auf ihre Anbindungsstärke an das Modellsubstrat getestet. Aufgrund des geringeren Absorptionsmaximums, im Vergleich zu dem bekannten BBG, wurden in diesem Fall 6 Konzentrationen von 0,005 mg/mL bis 0,16 mg/mL vermessen. Die Figuren 2 und 3 zeigen die graphischen Auftragungen der UV/Vis-spektroskopischen Messergebnisse.

Die Figuren 2 und 3 zeigen deutlich die sehr guten Anbindungen der Anthrachinonfarbstoffe **7b-d** an das Modellsubstrat. Da die Anthrachinonfarbstoffe zwei Absorptionsmaxima aufweisen, sind in den Grafiken zwei Referenz-Geraden sowie zwei Proben-Geraden aufgeführt. Die Absorptionsmaxima liegen bei 588 nm sowie 632-635 nm. Die Referenz-Absorptionsmaxima liegen dabei weit über denen der mit Modellsubstrat behandelten Werte. Höhere Konzentrationen konnten aufgrund der zu hohen Absorption der Referenz nicht aufgenommen werden. Tabelle 3 zeigt die Verhältnisse der λₘₐₓ-Werte der MS-Proben im Verhältnis zu den korrespondierenden Referenzen. Die genauen Messergebnisse der UV/Vis-spektroskopischen Untersuchungen befinden sich in den Tabellen 4 bis 7. Mit zunehmender Länge der Alkylreste steigt die Wechselwirkung der Farbstoffe mit dem Modellsubstrat. Während Verbindung **7a** mit Butylrest lediglich λₘₐₓ-Verhältnisse zwischen ca. 50 bis 80 % aufweist, können bei den Farbstoffen **7b-d** verbesserte Werte erzielt werden. Eindeutig ist jedoch festzuhalten, dass mit steigender Länge des Alkylrestes ebenfalls der Extinktionskoeffizient des Farbstoffes sinkt. Dies ist zum einen auf das Molekulargewicht der Verbindung, zum anderen auf den steigenden hydrophoben Charakter zurückzuführen.

**Tabelle 3: UV/Vis-spektroskopische Messergebnisse der 7-Referenzen und 7-MS-Lösung in Konzentrationen von 0,005 bis 0,16 mg/mL**

| | **7a** | | **7b** | | **7c** | | **7d** | |
|---|---|---|---|---|---|---|---|---|
| Konzentration [mg/mL] | Verhältnis λₘₐₓ mit MS und Referenz | | | | | | | |
| | 58 | 63 | 58 | 63 | 58 | 63 | 58 | 63 |
| | 8 nm | 2 nm | 8 nm | 4 nm | 8 nm | 5 nm | 8 nm | 4 nm |
| 0,005 | 54% | 54% | 11 % | 9% | 48% | 37% | 12% | 13% |
| 0,01 | 52% | 52% | 10% | 8% | 21 % | 17% | 7% | 8% |
| 0,02 | 54% | 55% | 6% | 6% | 7% | 7% | 3% | 4% |
| 0,04 | 57% | 58% | 8% | 8% | 4% | 5% | 3% | 3% |
| 0,08 | 67% | 69% | 18% | 19% | 8% | 10% | 1 % | 2% |
| 0,16 | 76% | 78% | 32% | 34% | 13% | 17% | 18% | 18% |

**Tabelle 4 UV/Vis-spektroskopische Messergebnisse der 7a-Referenzen und 7a-MS-Lösungen in Konzentrationen von 0,005 bis 0,16 mg/mL**

| | | | | | | |
|---|---|---|---|---|---|---|
| 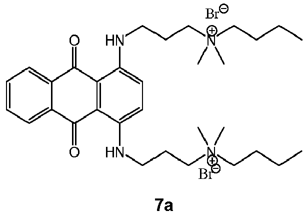 | | | | | | |

| | λₘₐₓ | λₘₐₓ | λₘₐₓ | λₘₐₓ | Verhältnis λₘₐₓ mit | |
|---|---|---|---|---|---|---|
| Konzentration | Referenz | mit MS | Referenz | mit MS | MS und Referenz | |
| [mg/mL] | (588 nm) | (588 nm) | (632 nm) | (632 nm) | 588 nm | 632 nm |
| 0,005 | 0,0932 | 0,0502 | 0,1159 | 0,0624 | 54% | 54% |
| 0,01 | 0,2000 | 0,1032 | 0,2448 | 0,1268 | 52% | 52% |
| 0,02 | 0,3868 | 0,2103 | 0,4640 | 0,2560 | 54% | 55% |
| 0,04 | 0,7611 | 0,4333 | 0,8862 | 0,5177 | 57% | 58% |
| 0,08 | 1,500 | 1,0086 | 1,6571 | 1,1524 | 67% | 69% |
| 0,16 | 2,8594 | 2,1625 | 2,9961 | 2,3477 | 76% | 78% |

**Tabelle 5 UV/Vis-spektroskopische Messergebnisse der 7b-Referenzen und 7b-MS-Lösungen in Konzentrationen von 0,005 bis 0,16 mg/mL**

| | | | | | | |
|---|---|---|---|---|---|---|
| 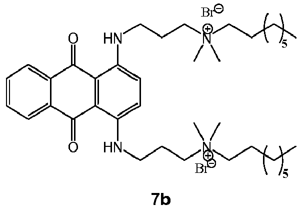 | | | | | | |

| | λₘₐₓ | λₘₐₓ | λₘₐₓ | λₘₐₓ | Verhältnis λₘₐₓ mit | |
|---|---|---|---|---|---|---|
| Konzentration | Referenz | mit MS | Referenz | mit MS | MS und Referenz | |
| [mg/mL] | (588 nm) | (588 nm) | (634 nm) | (634 nm) | 588 nm | 634 nm |
| 0,005 | 0,0733 | 0,0078 | 0,0930 | 0,0082 | 11 % | 9% |
| 0,01 | 0,1508 | 0,0150 | 0,1890 | 0,0159 | 10% | 8% |
| 0,02 | 0,3142 | 0,0201 | 0,3881 | 0,0232 | 6% | 6% |
| 0,04 | 0,6307 | 0,0496 | 0,7606 | 0,0609 | 8% | 8% |
| 0,08 | 1,2728 | 0,2326 | 1,4791 | 0,2873 | 18% | 19% |
| 0,16 | 2,4402 | 0,7719 | 2,6985 | 0,9204 | 32% | 34% |

**Tabelle 6 UV/Vis-spektroskopische Messergebnisse der 7c-Referenzen und 7c-MS-Lösungen in Konzentrationen von 0,005 bis 0,16 mg/mL**

| | | | | | | |
|---|---|---|---|---|---|---|
| 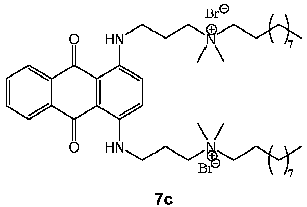 | | | | | | |

| | λₘₐₓ | λₘₐₓ | λₘₐₓ | λₘₐₓ | Verhältnis λₘₐₓ mit | |
|---|---|---|---|---|---|---|
| Konzentration | Referenz | mit MS | Referenz | mit MS | MS und Referenz | |
| [mg/mL] | (588 nm) | (588 nm) | (635 nm) | (635 nm) | 588 nm | 635 nm |
| 0,005 | 0,0503 | 0,0243 | 0,0633 | 0,0234 | 48% | 37% |
| 0,01 | 0,124 | 0,0267 | 0,1476 | 0,0261 | 21 % | 17% |
| 0,02 | 0,2708 | 0,0205 | 0,2786 | 0,0216 | 7% | 7% |
| 0,04 | 0,5298 | 0,0218 | 0,5068 | 0,0257 | 4% | 5% |
| 0,08 | 1,0636 | 0,0799 | 0,9575 | 0,0977 | 8% | 10% |
| 0,16 | 2,2754 | 0,2842 | 1,9492 | 0,3300 | 13% | 17% |

**Tabelle 7: UV/Vis-spektroskopische Messergebnisse der 7d-Referenzen und 7d-MS-Lösungen in Konzentrationen von 0,005 bis 0,16 mg/mL**

| | | | | | | |
|---|---|---|---|---|---|---|
| 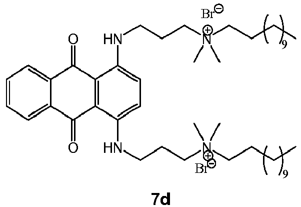 | | | | | | |

| | λₘₐₓ | λₘₐₓ | λₘₐₓ | λₘₐₓ | Verhältnis λₘₐₓ mit | |
|---|---|---|---|---|---|---|
| Konzentration | Referenz | mit MS | Referenz | mit MS | MS und Referenz | |
| [mg/mL] | (588 nm) | (588 nm) | (634 nm) | (634 nm) | 588 nm | 634 nm |
| 0,005 | 0,0369 | 0,0043 | 0,0304 | 0,0041 | 12% | 13% |
| | | | | | | |
| 0,01 | 0,1045 | 0,0074 | 0,0845 | 0,0071 | 7% | 8% |
| 0,02 | 0,2440 | 0,0081 | 0,1949 | 0,0080 | 3% | 4% |
| 0,04 | 0,3649 | 0,0101 | 0,2895 | 0,0100 | 3% | 3% |
| 0,08 | 0,8039 | 0,0120 | 0,6368 | 0,0120 | 1 % | 2% |
| 0,16 | 1,8155 | 0,3218 | 1,4306 | 0,2580 | 18% | 18% |

### c) UV/Vis-Messungen des Vitalfarbstoffs Indocyaningrün (ICG)

*Indocyaningrün* ist einer der wichtigsten Farbstoffe in der Chromovitrektomie, insbesondere im Bereich des "ILM-Peelings". Der Farbstoff wirft jedoch einige Problematiken sowie Schwierigkeiten in der Handhabung auf. Obwohl er lange Zeit für die Anfärbung der *membrana limitans interna* sowie epiretinalen Membranen eingesetzt wurde, konnten Studien, zahlreiche Nachteile des Farbstoffes aufzeigen. Neben postoperativen Verschlechterungen des Sehvermögens ist ebenfalls die geringe Stabilität unter Lichteinfluss ein gravierendes Problem bei Arbeiten mit dem Farbstoff. Zusätzlich kann ICG nicht in phosphatgepufferter Salzlösung eingesetzt sowie vermessen werden, da dies ein Ausfallen der Verbindung bewirkt. Aus diesem Grund mussten auch die nachfolgenden Messungen in doppelt destilliertem Wasser durchgeführt werden, da Messungen in Pufferlösung lediglich ein Absetzen des Farbstoffes nach Zentrifugieren zeigten. Die graphisch aufgetragenen UV/Vis-Messergebnisse sind in Figur 4 gezeigt.

Wie anhand der dargestellten Messergebnisse deutlich wird, zeigte ICG auch bei den vorliegenden Messungen einige Problematiken. Während der Farbstoff bis zu einer Konzentration von 0,02 mg/mL kaum eine Absorption aufwies, erreicht *Indocyaningrün* bei einer Konzentration von 0,04 mg/mL bereits eine Absorption von 3,5822 und somit den maximal möglichen Messwert. Eine höhere Konzentration konnte aufgrund der zu starken Absorption und damit ungenauer Messung nicht mehr festgehalten werden. Wie Tabelle 8*Tabelle* zu entnehmen ist, konnten die λₘₐₓ-Werte der Referenz und der Modellsubstrat-Farbstoff-Lösung zwar ins Verhältnis gesetzt werden und ergaben positive Anbindungen der Farbstoffes an das Modellsubstrat, jedoch sind die Ergebnisse durch die geringe Konzentration im Fehlerbereich starken Schwankungen unterlegen. Grund dafür könnte zum einen die schwache Absorption bei geringen Konzentrationen, zum anderen die Zersetzung durch Lichteinfluss sein. Während der durchgeführten Messmethode konnte ein vollständiger Ausschluss von Lichteinfluss nicht gewährleistet werden.

**Tabelle 8: UVlVis-spektroskopische Messergebnisse der ICG-Referenz und ICG-MS-Lösung in Konzentrationen von 0, 005 bis 0, 04 mg/mL**

| Konzentration [mg/mL] | λₘₐₓ Referenz (779 nm) | λₘₐₓ mit MS (780 nm) | Verhältnis λₘₐₓ mit MS und Referenz |
|---|---|---|---|
| 0,005 | 0,0226 | 0,0106 | 47% |
| 0,01 | 0,0162 | 0,0154 | 95% |
| 0,02 | 0,0477 | 0,0204 | 42% |
| 0,04 | 3,5822 | 3,0389 | 85% |

### Beispiel 8

Im folgenden werden einige Synthesebeispiele beschrieben und Schemata zur Herstellung von Farbstoffen gezeigt. Verwendet wurde dabei das 6-Methyl-substituierte Derivat des 1,4-Difluoranthrachinons, das ausgehend von 4-Methylphthalsäureanhydrid und 1,4-Difluorbenzol synthetisiert wurde und anschließend oxidiert werden sollte. Auf diese Weise kann die Modifizierung der Seitengruppen ohne mögliche Nebenreaktionen durch die Säure durchgeführt werden. Ebenso besteht dadurch die Möglichkeit einer vereinfachten Aufreinigung. Eine anschließende Oxidation der Methylgruppe in 6-Position sollte zur gewünschten Säuregruppe führen. Die Synthese des 1,4-Difluor-6-methylanthrachinons (14) ist in Schema 1 gezeigt.

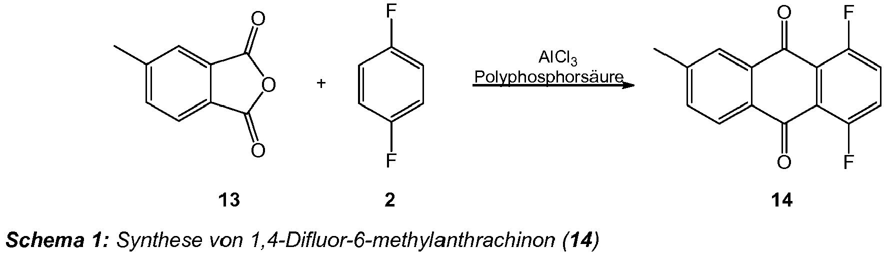

Die Reaktion wurde analog der Synthese des 1,4-Difluoranthrachinons (1) durchgeführt. Nach säulenchromatographischer Aufreinigung konnte die erfolgreiche Synthese der Verbindung 14 mittels 1H-NMR-, 13C-NMR- und IR-Spektroskopie, sowie Elementaranalyse in geringen Ausbeuten von ca. 20 % nachgewiesen werden. Im Folgenden wurde die Modifizierung mit N,N-Dimethylaminopropylamin (4) zum difunktionalisierten Produkt 1,4-Bis((3-(dimethylamino)propyl)amino)-6-methylanthrachinon (15) durchgeführt (Schema 2). Nach säulenchromatographischer Aufreinigung des Produktgemisches konnte Verbindung 15 mittels 1H-NMR-Spektroskopie eindeutig nachgewiesen werden. Die Fluor-benachbarten Protonen erzeugen auch in diesem Fall nach erfolgreicher Umsetzung zu Farbstoff 15 ein Singulett.

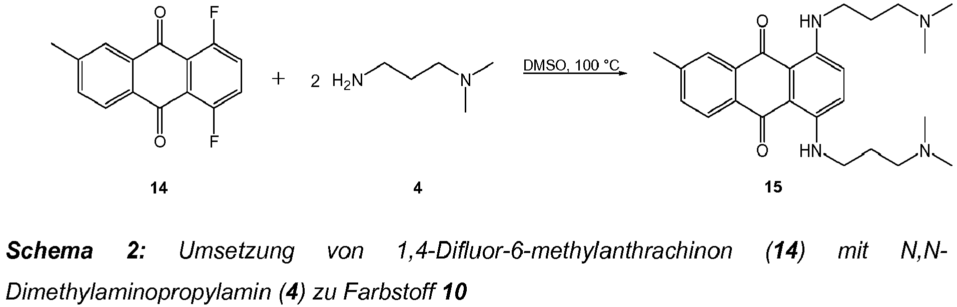

Aufgrund der zu diesem Zeitpunkt bereits bekannten höheren Selektivität des quartären Anthrachinonfarbstoffes mit C10-Rest wurde eine Quaternisierung des dimodifizierten 1,4-Difluor-6-methylanthrachinons 15 nur mit 1-Bromdecan durchgeführt. Schewma 3 zeigt die durchgeführte Reaktion.

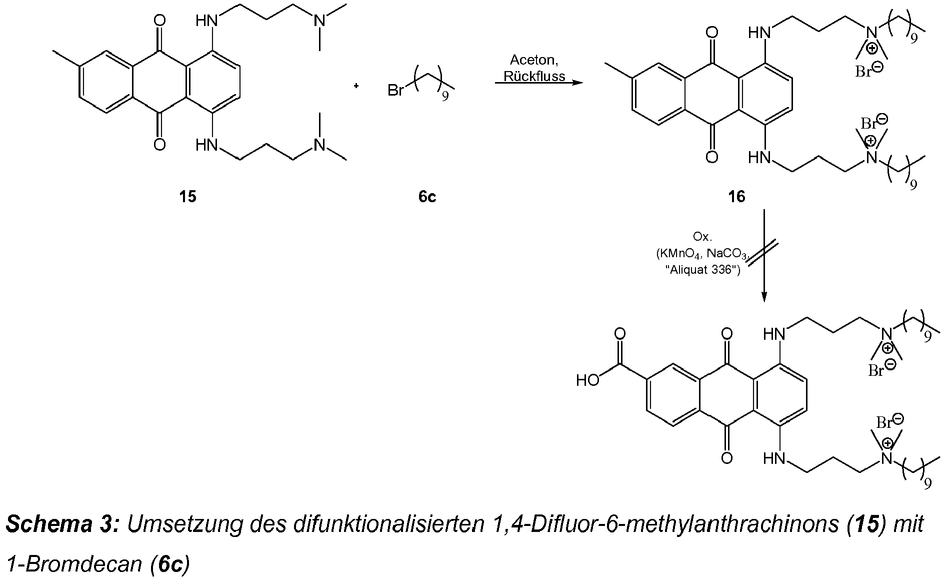

Die erfolgreiche Synthese des Anthrachinonfarbstoffes **16** konnte mittels ¹H-NMR-Spektroskopie eindeutig nachgewiesen werden. Die Verschiebung der Signale der amingebundenen Methylgruppen von 2,21 ppm zu 3,04 ppm spricht für eine erfolgreiche Synthese. Eine anschließende Oxidation der Methylgruppe von Verbindung 16 durch Kaliumpermanganat mit Natriumcarbonat, sowie dem Phasentransferkatalysator "Aliquat 336" in Dichlormethan:Wasser (1:1) konnte nicht erfolgreich durchgeführt werden. Aus diesem Grund wurde ein erneuter Versuch zur carboxylmodifizierten Anthrachinonverbindung direkt über Trimellitsäureanhydrid (17) und 1,4-Difluorbenzol (1) realisiert. Die durchgeführte Reaktion ist in Schema 4 gezeigt. Der Nachweis von 5,8-Difluor-9,10-dioxo-9,10-dihydroanthracen-2-carboxylsäure (18) erfolgte mittels 1H-NMR-Spektroskopie sowie Massenspektrometrie.

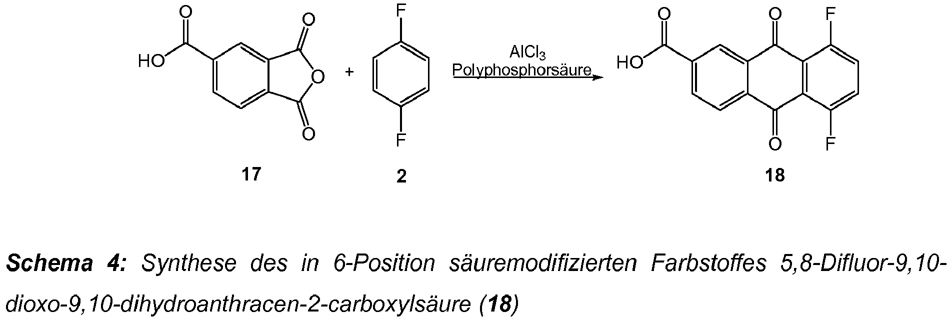

Die weitere Umsetzung zu 19 erfolgte analog vorangegangener Vorgehensweise durch Umsetzung mit N,N-Dimethylaminopropylamin (4) und ist in Schema 5 gezeigt.

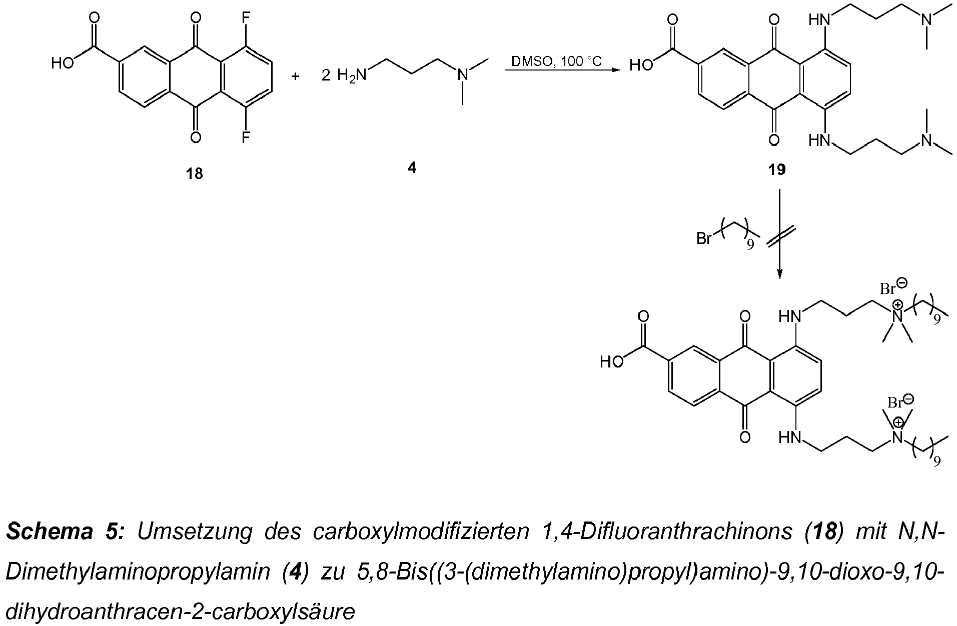

Die erfolgreiche Synthese des Anthrachinonfarbstoffes 5,8-Bis((3-(dimethylamino)propyl)amino)-9,10-dioxo-9,10-dihydroanthracene-2-carboxylsäure (19) ist durch 1H-NMR-spektroskopische sowie massenspektroskopische Analysen belegt. Der abschließende Schritt zum Einbau der positiven Ladung durch Umsetzung mit 1-Bromdecan konnte nicht erfolgreich durchgeführt werden. Zahlreiche Nebenreaktionen stören die Umsetzung und verhindern dadurch die Produktbildung. Ebenfalls ist eine anschließende Aufreinigung und Isolierung des Produktes durch die positive Ladung erschwert. Aus diesem Grund konnte die Zielstruktur nicht erfolgreich nachgewiesen werden.

### Beispiel 9 Polymere Anthrachinonfarbstoffe

Auf Basis von Verbindung N,N'-(((Anthrachinon-1,4-diyl)bis(azanediyl))bis(propane-3,1-diyl))bis(N,N-dimethyldecan-1-aminium)bromid (7c) sollten zusätzlich polymere Strukturen synthetisiert werden. Polymere verhindern bei Anwendungen im Körper eine Diffusion in tiefere Zellschichten und verhindern dadurch toxische Wirkungen. Aufgrund der hohen Reaktivität des Difluoranthrachinons gegenüber primären Aminen wurde Polyvinylamin (PVA) als polymere Grundstruktur verwendet. Zur Anbindung des Farbstoffes an das Polymer wurde deshalb ein monofunktionalisiertes Derivat eingesetzt, welches als Nebenprodukt bei der Synthese von Verbindung 5 entsteht und bei der säulenchromatographischen Aufreinigung sauber isoliert werden kann. Bei äquimolarem Einsatz der beiden Edukte kann analog Schema 6 Verbindung 20 in guten Ausbeuten isoliert werden.

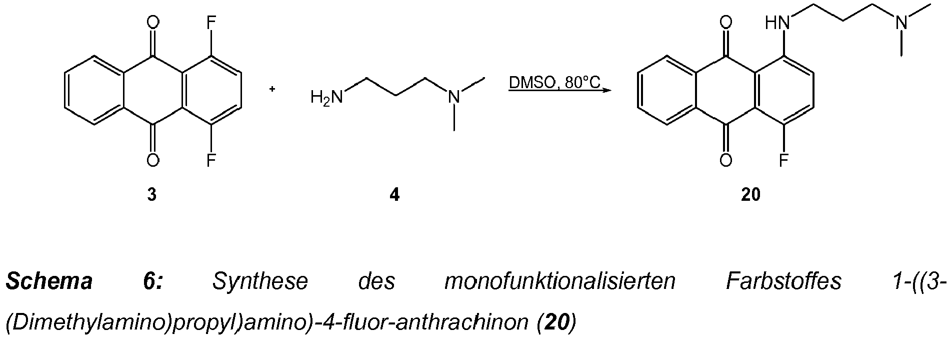

Die erfolgreiche Synthese der Verbindung **20** konnte mittels ¹H-NMR- und ¹³C-NMR-Spektroskopie nachgewiesen werden. Im Vergleich zum difunktionalisierten Derivat kann eindeutig gezeigt werden, dass die dem Fluor benachbarten aromatischen Protonen zwei Signale ergeben, da sie nicht chemisch äquivalent sind. Zusätzlich weisen die Signale eine höhere Multiplizität auf, da ebenfalls eine Kopplung mit dem Fluoratom beobachtet werden kann. Das dem Fluor-Atom benachbarte Wasserstoffatom zeigt im ¹H-NMR-Spektrum dabei zwei ³J-Kopplungen, während das zweite Wasserstoffatom sowohl eine ³J-Kopplung, als auch eine ⁴J-Kopplung erzeugt. Dies ermöglicht eine eindeutige Zuordnung der Signale.

Die weitere Umsetzung erfolgte analog zu den Verbindungen **7a-d**. Hierzu wurde der monofunktionalisierte Farbstoff **20** mit 1-Bromdecan (**6c**) in Aceton umgesetzt. Das Produkt fällt auch hier bei der Reaktion aus und kann mittels Filtration abgetrennt werden. Der Nachweis der erfolgreichen Synthese erfolgte mittels ¹H-NMR-Spektroskopie. Die Reaktion ist in Schema 7 gezeigt.

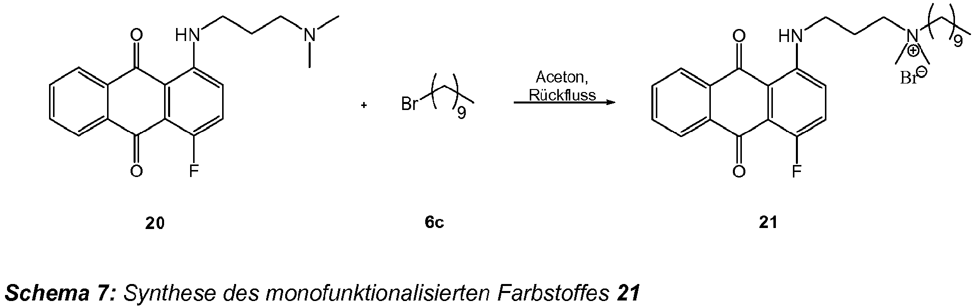

Die Anbindung an eine polymere Struktur wurde durch das freie Fluor-Atom der Verbindung **21** über die primären Amine des Polyvinylamins (PVA) realisiert. Die Umsetzung erfolgt in THF/H₂O mit Spuren von Triethylamin, um eine mögliche Protonierung der Aminogruppen zu verhindern. Das entstehende Polymer ist durch die polymeranaloge Anbindung an **21** über das Amin anschließend blau. Das Reaktionsschema ist im Folgenden gezeigt.

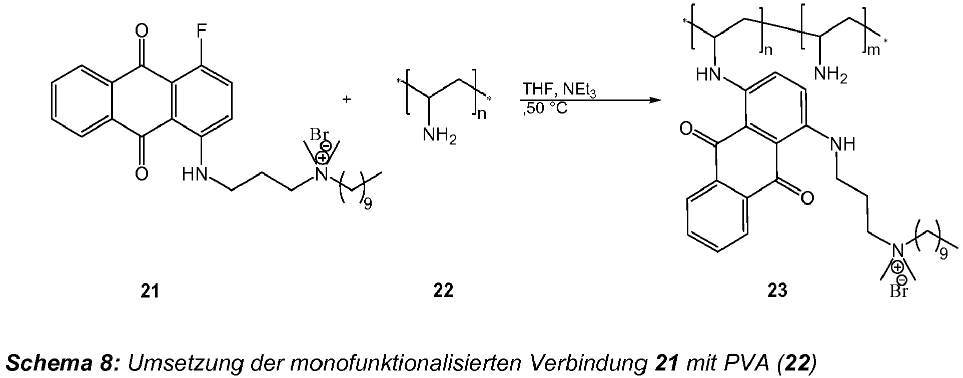

Der Anthrachinonfarbstoff wurde 10 und 20 Gew.-%ig in das Polymer eingebaut. Der Reaktionsverlauf ist aufgrund der Farbänderung durch die Substitution mittels bloßem Auge sowie mittels Dünnschichtchromatographie zu kontrollieren. Aufgrund des nicht vollständigen Umsatzes enthält das Polymer noch freie Aminogruppen, die zum einen eine Anbindung an das zu färbende Gewebe, zum anderen eine Molekulargewichtskontrolle über GPC stören. Deshalb wurde auf weitere analytische Methoden verzichtet. Die Evaluierung der Anbindungsstärke ist in Kapitel 3.1.2 gezeigt.

## Patentansprüche

1. Membranfärbekomplex umfassend
a) mindestens ein Targetingelement [A-L-Q⁺-Alk B⁻], worin A eine Ankergruppe, L ein Linker, Q⁺ eine quaternäre Ammoniumgruppe, B⁻ ein ophthalmologisch annehmbares Gegenion und Alk eine Alkylkette mit einer Kettenlänge von 4 bis 12 Kohlenstoffatomen bedeutet;
b) mindestens ein Chromophor und gegebenenfalls
c) ein Trägermolekül.

2. Membranfärbekomplex nach Anspruch 1, **dadurch gekennzeichnet, dass** die Ankergruppe ein Heteroatom oder eine funktionelle Gruppe ist.

3. Membranfärbekomplex nach Anspruch 1 oder Anspruch 2, **dadurch gekennzeichnet, dass** die Ankergruppe eine Aminogruppe -(R¹)N-. worin R¹ H oder eine C₁-C₄-Alkylgruppe ist, und der Linker -C₁-C₆-Alkyl- ist,.

4. Membranfärbekomplex nach einem der vorhergehenden Ansprüche, **dadurch gekennzeichnet, dass** -Q⁺- -(R²)(R³)N⁺- ist, worin R² und R³ jeweils unabhängig voneinander C₁-C₄-Alkylreste sind.

5. Membranfärbekomplex nach einem der vorhergehenden Ansprüche, **dadurch gekennzeichnet, dass** das Gegenion B- ein Halogenidion, Hydrogenphosphation oder Hydrogensulfation ist.

6. Membranfärbekomplex nach einem der vorhergehenden Ansprüche, **dadurch gekennzeichnet, dass** das Chromophor ein ophthalmologisch verträglicher wasserlöslicher Farbstoff ist.

7. Membranfärbekomplex nach einem der vorhergehenden Ansprüche, **dadurch gekennzeichnet, dass** das Chromophor eine Verbindung der Formel I 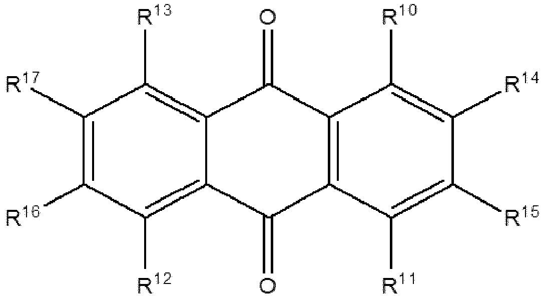 ist, wobei jeder der Reste R¹⁰, R¹¹, R¹² und R¹³ jeweils unabhängig voneinander Wasserstoff, C₁-C₄-Alkylreste, eine Ankergruppe, wie in Anspruch 1 definiert, oder ein Linkermolekül ist,
jeder der Reste R¹⁴, R¹⁵, R¹⁶ und R¹⁷ jeweils unabhängig voneinander Wasserstoff, ein C₁-C₄-Alkylrest oder eine Linkereinheit ist.

8. Membranfärbekomplex nach einem der vorhergehenden Ansprüche, **dadurch gekennzeichnet, dass** das Chromophor eine Verbindung der Formel I ist, worin R¹⁰ und R¹¹ jeweils eine Targeteinheit ist und R¹², R¹³, R¹⁴, R¹⁵, R¹⁶ und R¹⁷ jeweils Wasserstoff sind.

9. Membranfärbekomplex nach Anspruch 6, **dadurch gekennzeichnet, dass** R¹⁰ und R¹¹ jeweils eine Targeteinheit ist, einer der Reste R¹², R¹³, R¹⁴, R¹⁵, R¹⁶ und R¹⁷ eine Linkereinheit ist, und die restlichen Reste jeweils Wasserstoff sind.

10. Membranfärbekomplex nach einem der vorhergehenden Ansprüche, **dadurch gekennzeichnet, dass** der Komplex ein Trägermolekül aufweist, wobei an dem Trägermolekül mindestens ein Chromophor und mindestens ein Targetingelement gebunden sind.

11. Membranfärbekomplex nach einem der vorhergehenden Ansprüche, **dadurch gekennzeichnet, dass** Targetingelement und Chromophor jeweils unabhängig voneinander an das Trägermolekül gebunden sind.

12. Membranfärbekomplex nach einem der Ansprüche 1 bis 9, **dadurch gekennzeichnet, dass** mindestens ein Targetingelement an ein Chromophor gebunden ist, wobei das Trägermolekül Chromophore und Targetingelemente unabhängig voneinander und/oder Chromophore mit daran gebundenen Trägerelementen gebunden aufweist.

13. Membranfärbekomplex nach einem der vorhergehenden Ansprüche, **dadurch gekennzeichnet, dass** das Trägermolekül ein wasserlösliches ophthalmologisch annehmbares Polymer ist, das funktionelle Gruppen zur Bindung von Chromophor- und/oder Targeteinheiten aufweist.

14. Membranfärbekomplex nach einem der vorhergehenden Ansprüche, **dadurch gekennzeichnet, dass** das Trägermolekül ein lineares, verzweigtes oder beadartiges wasserkompatibles Polymer ist.

15. Membranfärbekomplex nach einem der vorhergehenden Ansprüche, **dadurch gekennzeichnet, dass** das Trägermolekül ein von Vinylamin abgeleitetes Homo- oder Copolymer ist.

16. Verwendung eines Membranfärbekomplexes nach einem der Ansprüche 1 bis 13 zur Anfärbung von okularen Membranen, wie epiretinalen Membranen (ERM) oder der Membrana limitans interna (ILM).
